# EUROPEAN PATENT APPLICATION

(11) **EP 4 371 613 A2**
(43) Date of publication of application: **22.05.2024**
(21) Application number: 24166426.7
(22) Date of filing: 26.02.2019
(51) Int. Cl.: A61P 27/02

(54) **COMPOUNDS FOR USE IN THE TREATMENT OF OCULAR PAIN**

(30) Priority: 26.02.2018 EP 18158674
(62) Divisional of application: 19706664.0
(71) Applicant: Ospedale San Raffaele S.r.l., 20132 Milano (IT)
(72) Inventor: RAMA, Paolo, 20132 Milano (IT); FERRARI, Giulio, 20132 Milano (IT)
(74) Representative: Pace Napoleone, Maria

(57) **Abstract**

The invention relates to compounds, in particular NK-1 antagonists, for use in the treatment of ocular sensitivity and/or ocular pain.

## Description

### FIELD OF THE INVENTION

The invention relates to compounds, in particular NK-1 antagonists, for use in the treatment of ocular sensitivity and/or ocular pain.

### BACKGROUND TO THE INVENTION

The problem of ocular pain has been largely underestimated for decades. Recently, however, it has been object of revived interest, and its elevated prevalence has finally been acknowledged¹. Virtually any ocular surface disease or surgery will induce corneal pain at some level. Ocular pain is a consequence of highly prevalent ocular surface diseases (keratitis, conjunctivitis, blepharitis, corneal edema and dry eye²⁻⁵), surgery (refractive surgery, corneal crosslinking, keratoplasty, cataract and retina surgery⁶⁻⁹,) and in-office clinical procedures like contact lenses wearing¹⁰. Sometimes, corneal pain can be excruciating to the point that affected patients have attempted suicide¹¹.

Importantly, it has now become apparent that ocular pain can be not only a consequence of other disorders, but also an autonomous disease entity, where treatment remains sub-optimal¹².

Pain of the ocular surface is a common disorder associated with almost any affections of the external eye, including ocular surface diseases, ocular surgeries and minor ocular procedures. Ocular surface diseases associated with corneal pain include keratitis, conjunctivitis, blepharitis, uveitis, corneal edema, dry eye, bullous keratopathy, ocular trauma/injuries, photo refractive keratotomy, radial keratotomy, contact lens intolerance. Common ocular procedures associated with ocular pain include refractive surgery, corneal crosslinking, cataract surgery, keratoplasty, glaucoma and retina surgery. Clinical procedures commonly used in ophthalmology may be associated with ocular surface pain and include: Goldman tonometry, gonioscopy, contact lens application for diagnostic purposes (three mirror lens, for instance), or for therapeutic purposes (e.g. laser photocoagulation of the retina).

Although vastly underestimated in the past decades, ocular pain has been recently subject to revived interest (PMID: 28736339), and its elevated prevalence finally acknowledged. Ocular pain significantly impairs the quality of life of affected patients. This is not surprising since the ocular surface receives the densest sensory innervation of the entire body. Indeed, ocular pain can be extremely disabling, and suicide attempts have been reported as its consequence (PMID: 23664666).

For these reasons, ocular pain is an area of significant and unmet medical need, and a major medical challenge. Current treatments include topical anesthetics (e.g oxybuprocaine chloride), topical non-steroidal anti-inflammatory drugs (NSAIDs) and systemic analgesics¹³.

All of these options are not completely satisfactory due to interference in wound healing processes (anesthetics, NSAIDs), gastrointestinal, kidney and liver toxicity, or central nervous system depression (systemic analgesics).

Topical anesthetics (e.g. oxybuprocaine chloride, 4mg/ml) are highly effective, but they can only be administered for limited time and are associated with significant side effects, such as a relatively short duration of action and imparing the wound healing process. Moreover, surprisingly, their use does not seem to be associated with significant analgesia in pain control of corneal abrasions (PMID:28480151), a common cause of ocular pain. In addition, it is well known that their prolonged use induces toxic keratopathy, corneal melting and perforation, which make their safety profile unacceptable by many authors (PMID:27347060, 15220742, 17662448, 9818348). Finally, a recent meta-analysis did not find any significant improvement in symptoms and pain in patients treated with topical anesthetics versus placebo (PMID: 26472608).

Topical NSAIDs have also been proposed for the treatment of corneal pain. They are generally less effective than topical anesthetics, and a recent Cochrane meta-analysis failed to provide strong evidence supporting the use of NSAIDs in corneal abrasions (a common cause of ocular pain (PMID:28516471). Others found some efficacy, although the limited sample size of the studies made it difficult to draw definitive conclusions (PMID: 28480151). As for the safety profile, delayed wound healing has been described and is associated with the use of topical NSAIDs (PMID: 25263042). It is generally convened that NSAIDs can negatively affect corneal wound healing, since their use is associated with corneal melting/perforation (PMID: 11797308, 16371788, 11320025).

Systemic analgesics are effective although their use is associated with significant systemic side effects (gastro-intestinal, liver and kidney toxicity, reduced alertness, hallucinations). As a general note, moreover, when the pain is limited to the ocular surface, there is limited rationale for using systemic pain control, when topical alternatives are available.

Therefore, there is still the need for a medicament for use in ocular pain prevention and therapy which is devoid of the disadvantages associated with current therapies.

Substance P (SP) is a C-amidated decapeptide that belongs, along with neurokinin-A, neurokinin-B, and neuropeptide-K, to the tachykinin family. The tachykinin receptor system belongs to the GPCR superfamily and comprises three subtypes of receptors, namely NK1, NK2, NK3. The principal receptor for substance P is NK-1.

Substance P is abundantly expressed in central, peripheral, and enteric nervous systems. It is also present in the peripheral sensory nerves of the cornea¹⁸.

Substance P concentration is increased in animal models of ocular diseases commonly associated with pain, such as alkali burn, intrastromal suture placement.

SP exerts its action by binding to its main Neurokinin 1 receptor (NK1-R). Binding is followed by internalization, and eventually results in neuron sensitization and activation of specific neurons in the spinal cord^{38,39}. The mechanisms controlling pain regulation of SP and NK1 in the trigeminal nerve are, however, not clear.

Over 300 patents have been filed in the past two decades in the NK1 antagonist field⁴⁰, with compounds under investigation and development for various diseases, from depression to cancer.

Fosaprepitant (Ivemend) is a water soluble phosphorylated pro-drug of Aprepitant, a highly selective Neurokinin 1 receptor antagonist¹⁴. In the last decade, Fosaprepitant has been effectively used against nausea and vomiting in both acute and delayed phases of chemotherapy^{15,16}. It is then clinically approved in the US and EU for the treatment of acute and delayed nausea and vomit associated with chemotherapy by intra-venous administration. Its water solubility makes it an ideal candidate to be tested for topical eye delivery. Its long clinical use warrants an excellent safety profile.

WO 2013/004766 describes NK-1 receptor antagonists and relative pharmaceutical compositions for use in the treatment or prevention of corneal neovascularisation. WO 2013/004766 does not refer to the treatment and/or prevention of ocular sensitivity and/or ocular pain.

WO 98/14193 describes the use of a substance P antagonist for the treatment of ocular pain and compositions for topical ocular use wherein the antagonist has the following formula.

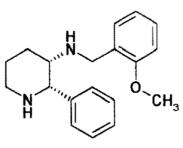

There is still the need for effective treatment for ocular pain, especially for topical formulations.

### SUMMARY OF THE INVENTION

Ocular surface pain is highly prevalent and invalidating. Current treatments remain sub-optimal and are associated with significant side effects.

In the present invention the effect of Fosaprepitant, a selective neurokinin-1 receptor (NK1R) antagonist, on trigeminal nociception in mice *and* in one patient affected with severe ocular pain was observed.

In WT mice, corneal nociception before and after different topical application of Fosaprepitant was measured. In B6.Cg-Tac1^{tm1Bbm}/J mice (SP-KO) mice, whether ablation of the SP is associated with reduced ocular surface pain was tested. In addition, the expression of NK1R in corneal nerves on mouse and human corneal whole-mounts was assessed. Finally, one patient affected with severe ocular pain in ocular pemphigoid was treated with topical Fosaprepitant (10mg/ml); subjective pain score and corneal sensitivity were measured.

It was observed that repeated 2 mg/ml or single 10 mg or 50 mg/ml Fosaprepitant topical administration induce corneal analgesia in WT mice. Fosaprepitant induced less pronounced analgesia than Diclofenac or Oxybuprocaine. The reduction of corneal nociception in treated WT mice approached the difference observed between SP-KO and WT mice. The expression of NK1R on corneal nerves was observed, suggesting that Fosaprepitant may act directly *in situ,* on nerve fibers.

Finally, topical instillation of Fosaprepitant (10mg/ml) reduced ocular pain and corneal sensitivity in the treated patient.

Then, it was surprisingly found that topical inhibition of NK1 receptor by means of highly selective NK1 antagonists reduces corneal nerve sensitivity and corneal pain.

In particular, the present data also support Fosaprepitant as a useful tool for the treatment and/or prevention of ocular surface pain.

Fosaprepitant, preferably at a specific concentration of at least 10mg/ml, is particularly effective.

In addition, the present invention shows that NK1 receptor-knock-out mice exhibit reduced corneal nerve sensitivity, which supports a key role of NK1-R in the generation/maintenance of corneal pain.

It is therefore an object of the invention an NK-1 antagonist for use in the prevention and/or the treatment of ocular pain and/or ocular sensitivity.

Preferably the NK-1 antagonist is Fosaprepitant or a pharmaceutically acceptable salt thereof.

Preferably the NK-1 antagonist is administered at a concentration of at least 1 mg/ml, preferably at least 10 mg/mL, preferably of at least 20 mg/mL, preferably of at least 30 mg/mL, preferably of at least 40 mg/mL, preferably of at least 50 mg/mL, preferably of at least 60 mg/mL, preferably at least 100 mg/mL.

Preferably the NK-1 antagonist is administered once at a concentration of approximately 50 mg/mL.

Preferably the NK-1 antagonist is administered once at a concentration of approximately 10 mg/mL.

Preferably the NK-1 antagonist is administered between once and six times a day. Preferably the NK-1 antagonist is administered for 1 day to 6 months, preferably for 1 day to 30 days at a concentration of 1 to 100 mg/ml.

Preferably the ocular sensitivity and/or ocular pain is caused by any one of the following conditions or pathology: keratitis, conjunctivitis, blepharitis, uveitis, corneal edema, dry eye, bullous keratopathy, ocular trauma/injuries, photo refractive keratotomy, radial keratotomy, contact lens intolerance, glaucoma, inflammatory disorders such as ocular pemphigoid, atopic conjunctivitis, rosacea, graft rejection, Lyell's syndrome, Stevens-Johnson syndrome, graft versus host disease; infectious keratitis including viral keratitis such as keratitis caused by infection with herpes simplex or herpes zoster, viral interstitial keratitis, bacterial keratitis such as keratitis caused by infection with Pseudomonas (e.g. Pseudomonas Aeruginosa), *Chlamydia trachomatis,* Treponema pallidum), fungal keratitis such as keratitis caused by Candida, Fusarium and Aspergillus spp and parasitic keratitis such as keratits caused by Onchocerciasis; degenerative disorders including congenital disorders such as pterygium, Terrien's marginal degeneration and aniridia; traumatic disorders such as ulcerations, acid burns, alkali burns; trauma and/or ocular tissue disruption associated with medical or surgical procedures; disorders associated with extended contact lens wear; stem cell deficiency (e.g. of limbus), including idiopathic, traumatic, aniridia, autoimmune polyendocrinopathy, infections caused by *Staphylococcus, Streptococcus, Pseudomonas* or microbial keratoconjuctivitis, *Pseudomonas aeuruginosa* infection, chemical or physical insult of the eye, ocular surgery, minor ocular procedures, refractive surgery, corneal crosslinking, cataract surgery, keratoplasty, glaucoma surgery, retina surgery, Goldman tonometry, gonioscopy, contact lens application with the three mirror lens, laser photocoagulation of the retina and any procedure inducing temporary or permanent stimulation of the trigeminal nerve fibers reaching the eye.

Preferably the NK-1 antagonist is administered prior to a surgery to the eye, or post surgery wherein said administration controls pain/hypersensitivity.

The present invention also provides a pharmaceutical composition comprising an NK-1 antagonist and a pharmaceutically acceptable vehicle, for use in the prevention and/or the treatment of ocular sensitivity and/or ocular pain.

Preferably the NK-1 antagonist is Fosaprepitant or a pharmaceutically acceptable salt thereof.

Preferably the pharmaceutical composition comprises at least 1 mg/ml, preferably at least 10 mg/mL of NK-1 antagonist.

Preferably the pharmaceutical composition further comprises at least one agent selected from the group consisting of: an anaesthetic agent, a non-steroidal anti-inflammatory agent, an analgesic agent, an agent useful in the prevention and/or treatment of the disease or condition that causes the ocular sensitivity and/or ocular pain, and an agent that is used following surgery to the eye. In certain conditions, it may be advantageous to temporarily reduce corneal sensitivity with Fosaprepitant to reduce discomfort.

Preferably the pharmaceutical composition is for topical use.

Preferably the composition is a nanosome and/or liposome formulation
The present invention also provides an eye-drop comprising an NK-1 antagonist and a pharmaceutically acceptable vehicle, for use in the prevention and/or the treatment of ocular sensitivity and/or ocular pain

A preferred formulation is where fosaprepitant is administered topically in the conjunctival sac, or subconjunctivally, or systemically, preferably at a concentration of 1-100mg/ml, and administered from 1 to 10 times a day, preferably for 1 day to 6 months, preferably for 1 day to 30 days.

Preferably the NK-1 antogonist is administered topically in the conjunctival sac, or subconjunctivally, or systemically, preferably at a concentration of 1-100mg/ml, and administered from 1 to 10 times a day for 1 day to 6 months.

Preferred administration is administration into the anterior chamber, intravitreal injection, subretinal injection, parabulbar and/or retrobulbar injection, intrastromal corneal injection. As used herein, the expression "for use in the prevention and/or the treatment of ocular sensitivity and ocular pain" include the treatment, amelioration or prevention of conditions of the eye characterised by increased sensitivity and/or pain.

Ocular sensitivity is measured as increased number of mice wipings following instillation of NaCl solution into the conjunctival sac. Eye/ocular pain is measured as detailed in methods and with a method based on published evidence³⁶.

The treatment may be prophylactic; thus, the treatment may be administered to individuals at risk of acquiring the conditions described herein.

In particular, said increased sensitivity and/or pain may be caused by a disease or condition or by surgery, including a minor ocular procedure.

For instance, diseases or conditions that may be associated with increased sensitivity and/or pain, which may be treated according to the teachings of the present invention include but are not limited to: keratitis, conjunctivitis, blepharitis, uveitis, corneal edema, dry eye, bullous keratopathy, ocular trauma/injuries, photo refractive keratotomy, radial keratotomy, contact lens intolerance, glaucoma, inflammatory disorders such as ocular pemphigoid, atopic conjunctivitis, rosacea, graft rejection, Lyell's syndrome, Stevens-Johnson syndrome, graft versus host disease; infectious keratitis including viral keratitis such as keratitis caused by infection with herpes simplex or herpes zoster, viral interstitial keratitis, bacterial keratitis such as keratitis caused by infection with Pseudomonas (e.g. Pseudomonas Aeruginosa), *Chlamydia trachomatis,* Treponema pallidum), fungal keratitis such as keratitis caused by Candida, Fusarium and Aspergillus spp and parasitic keratitis such as keratits caused by Onchocerciasis; degenerative disorders including congenital disorders such as pterygium, Terrien's marginal degeneration and aniridia; traumatic disorders such as ulcerations, acid burns, alkali burns; trauma associated with medical or surgical procedures, ocular pain following surgical procedures; disorders associated with extended contact lens wear; stem cell deficiency (e.g. of limbus), including idiopathic, traumatic, aniridia, autoimmune polyendocrinopathy, infections caused by *Staphylococcus, Streptococcus, Pseudomonas* or microbial keratoconjuctivitis, *Pseudomonas aeuruginosa* infection, acanhameoba keratitis and acanthamoeba scleritis, fungal keratitis, viral keratitis and viral conjunctivitis, dry eye, endophthalmitis, blepharitis, ocular cellulitis, chemical or physical insult of the eye, cicatrizing conjunctivitis, surgical or traumatic scarring of the conjunctiva. Examples of surgeries, including minor ocular procedures, that may be associated with increased sensitivity and/or pain, which may be treated according to the teachings of the present invention include but are not limited to: refractive surgery, corneal crosslinking, cataract surgery, keratoplasty, glaucoma surgery, retina surgery, Goldman tonometry, gonioscopy, contact lens application with the three mirror lens, laser photocoagulation of the retina, and any procedure which temporarily or permanently stimulates trigeminal nerve terminations of the eye. In the present invention, the expression "NK-1 antagonists" refers to refers to any agent (chemical compound, siRNA, miRNA, ect...) which antagonizes in any possible way the biological activities exerted by the neurokinin receptor 1.

NK-1 antagonists for use in the present invention may, for example, have an inhibitory concentration (IC50) against the human NK-1 receptor in competition with substance P of less than 100 µM, preferably less than 10 µM, preferably less than 1 µM, preferably less than 100 nM, preferably less than 10 nM, as measured by radiolabeled ligand binding assay on human cells transfected with NK-1 receptor⁴¹.

NK-1 antagonists are suitably selective antagonists, NK-1 antagonists are suitably selective for NK-1 over other receptors, especially NK-2 and NK-3. Thus NK-1 antagonists may, for example, have an inhibitory concentration (IC50) against the human NK-2 receptor in competition with Neurokinin A which is at least 10 times greater than the inhibitory concentration (IC50) against the human NK-1 receptor in competition with substance P (i.e. it is at least 10 fold selective for NK1 over NK-2), preferably at least 50 fold, preferably at least 100 times selective for NK-1 over NK-2. NK-1 antagonists may, for example, have an inhibitory concentration (IC50) against the human NK-3 receptor in competition with Neurokinin B which is at least 10 times greater than the inhibitory concentration (IC50) against the human NK-1 receptor in competition with substance P (i.e. it is at least 10 fold selective for NK1 over NK-3), preferably at least 50 times, preferably at least 100 fold selective for NK-1 over NK-3.

IC50 values against NK-1, NK-2 and NK-3 receptors may be determined by radiolabelled ligand binding assay of human cells transfected with NK1, NK2 or NK3 receptors⁴¹.

Preferably, the NK-1 antagonist for use in the present invention is selected from the list of NK-1 antagonists disclosed in WO 2013/004766, which is herein incorporated by reference in its entirety.

In particular, the NK-1 antagonist for use in the present invention may be selected from the list consisting of:
a. Aprepitant (MK-0869L-754,030), IUPAC name 5-([(2R,3S)-2-((R)-1-[3,5-bis(trifluoromethyl)phenyl]ethoxy)-3-(4-fluorophenyl)morpholino]methyl)-1H-1,2,4- triazol-3(2H)-one, 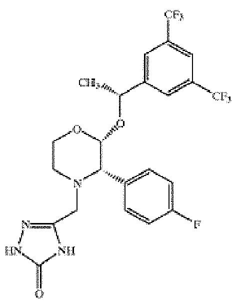 as described and claimed in the following US patents: US 5,719,147, US 5,538,982, US 6,048,859, US 6,096,742 and US 6,235,735, the contents of which are incorporated herein by reference in their entirety. Also described in reference 42; as well as pro-drugs thereof, such as:
   Fosaprepitant (L-758,298, Emend) IUPAC name [3-{[(2R,3S)-2-[(1R)-1-[3,5-bis(trifluoromethyl)phenyl]ethoxy]-3-(4-fluorophenyl)morpholin-4-yl]methyl}-5-oxo- 2H-1,2,4-triazol-1-yl]phosphonic acid 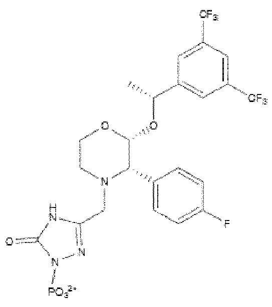
   e.g. in the form of a salt such as the dimeglumine salt
   as described and claimed at least in US 5,691,336, the contents of which are incorporated herein by reference in its entirety;
b. ZD4974 as described in WO02026724 and WO01077089, the contents of which are incorporated herein by reference in its entirety: 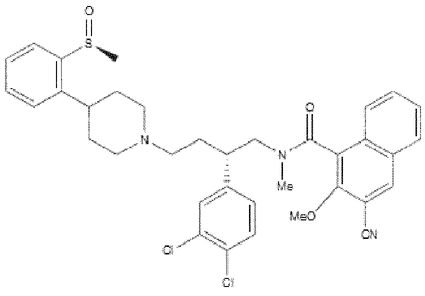
c. The following compound, described in WO01077069 and WO00059873, the contents of which are incorporated herein by reference in entirety: 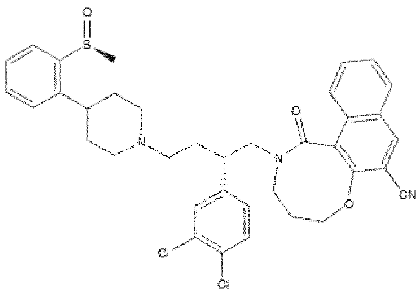
d. The following compound described in DE19519245, the contents of which are incorporated herein by reference in its entirety: 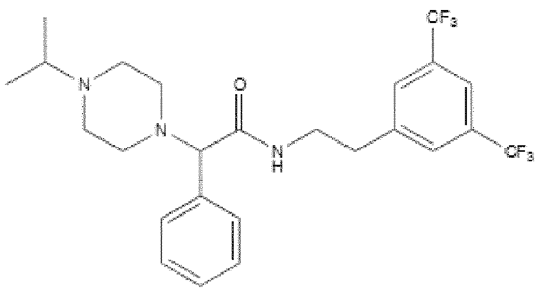
e. The following compound described in WO9732865, the contents of which are incorporated herein by reference in its entirety: 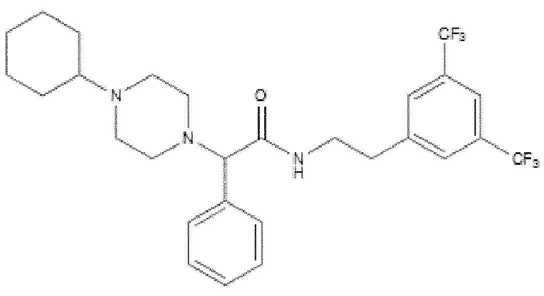
f. The following compound described in EP1295599, the contents of which are incorporated herein by reference in its entirety: 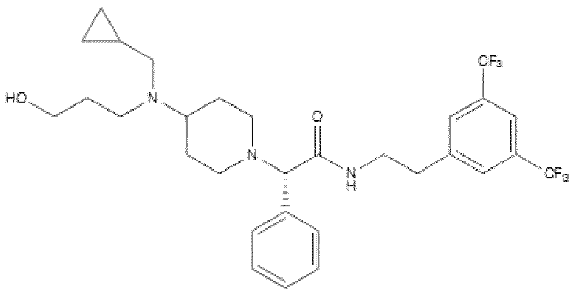
g. CGP49823 described in WO9626183 and WO9610562, the contents of which are incorporated herein by reference in their entirety: 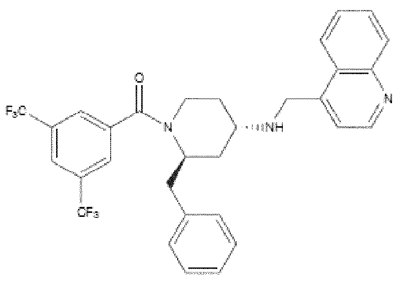
h. The following compound as described in WO9514017, the contents of which are incorporated herein by reference in its entirety: 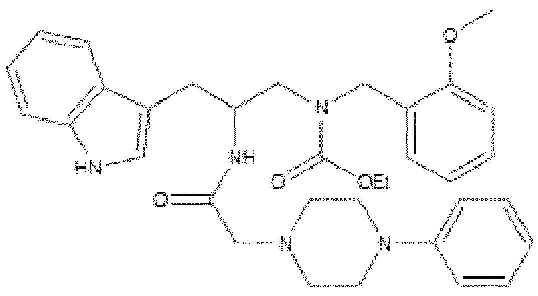
i. LY303870, Lanepitant, described in WO9907681, the contents of which are incorporated herein by reference in its entirety: 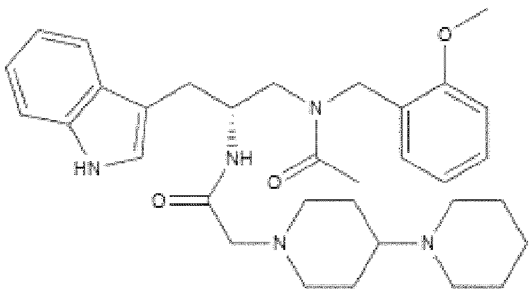
j. LI 686017, described in WO03091226, the contents of which are incorporated herein by reference in its entirety: 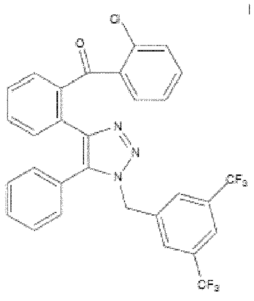
k. FK888, as described in ref 43 and WO9222569, WO93141 13, WO9321215, EP655442 and WO9637488, the contents of which are incorporated herein by reference in their entirety: 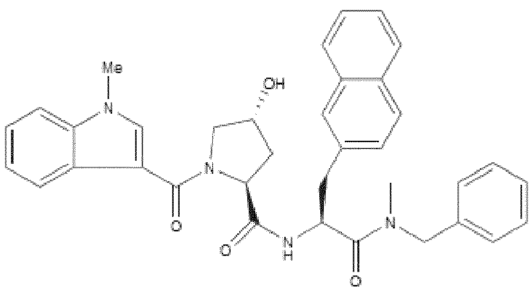
l. The following compound, described in WO9222569, WO93141 13, WO9321215, EP655442 and WO9637488, the contents of which are incorporated herein by reference in their entirety: 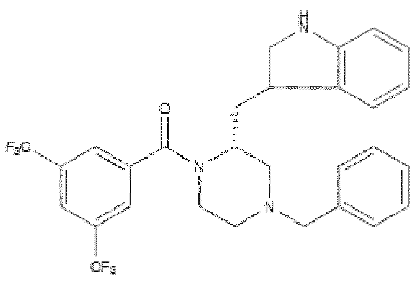
m. The following compound, described in WO9222569, WO9314113, WO9321215, EP655442 and WO9637488, the contents of which are incorporated herein by reference in their entirety: 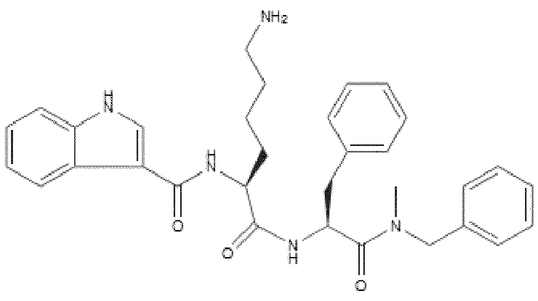
n. The following compound, described in WO00053572, the contents of which are incorporated herein by reference in its entirety: 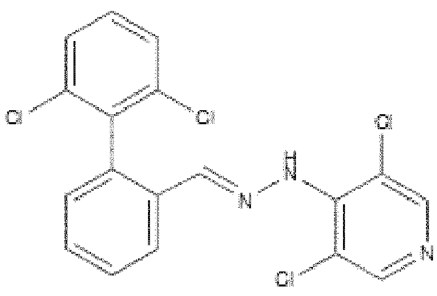
o. Netupitant, described in WO020008232, the contents of which are incorporated herein by reference in its entirety: 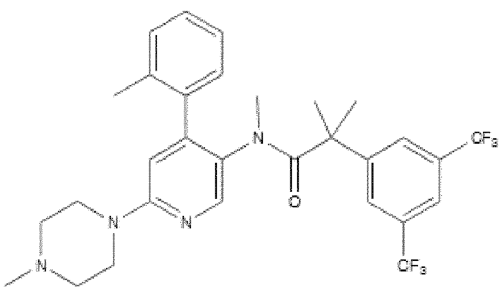
p. Befetupitant, described in WO020008232, the contents of which are incorporated herein by reference in its entirety: 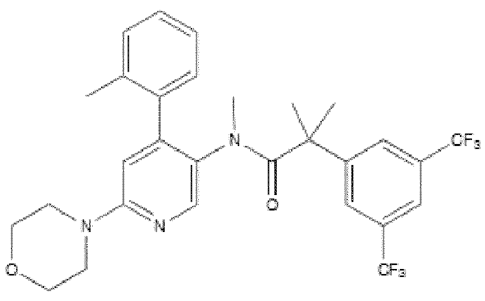
q. The following compound, described in WO202062784 and WO020008232, the contents of which are incorporated herein by reference in their entirety: 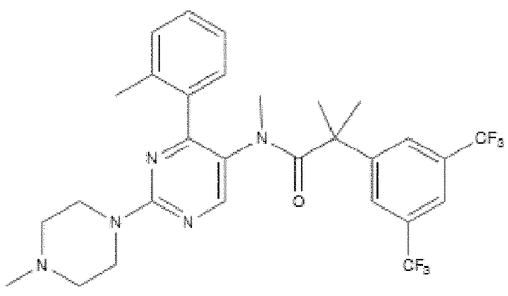
r. R116031, described in WO9724356 and WO0716440, the contents of which are incorporated herein by reference in their entirety: 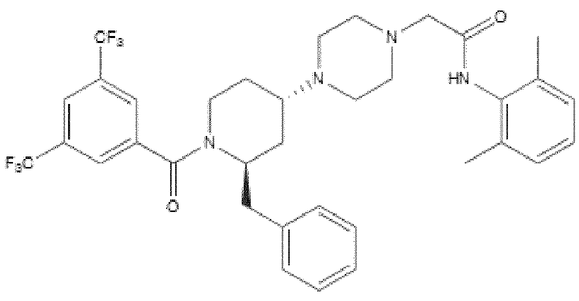
s. The following compound, described in EP522808, the contents of which are incorporated herein by reference in its entirety: 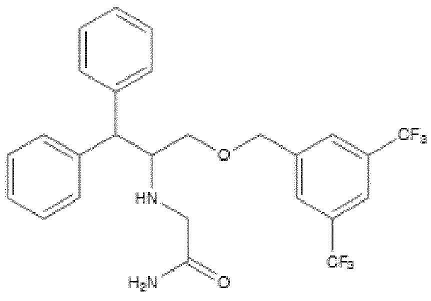
t. The following compound: 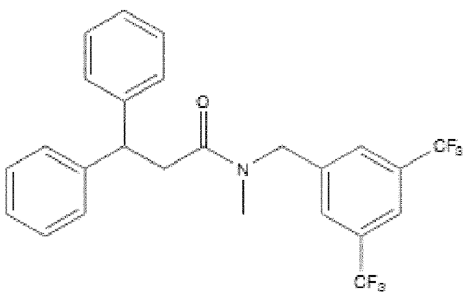
u. L733,060 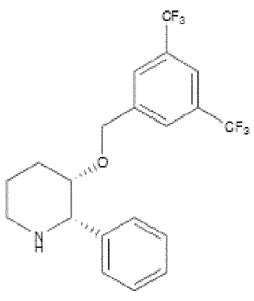
v. L736,281 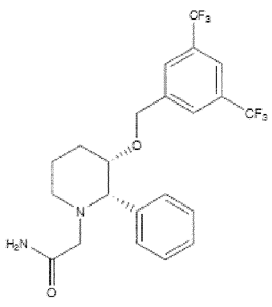
w. TKA731, described in WO9831704, the contents of which are incorporated herein by reference in its entirety: 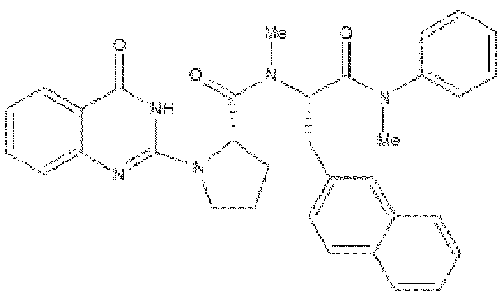
x. NKP608, described in WO04024714, the contents of which are incorporated herein by reference in its entirety: 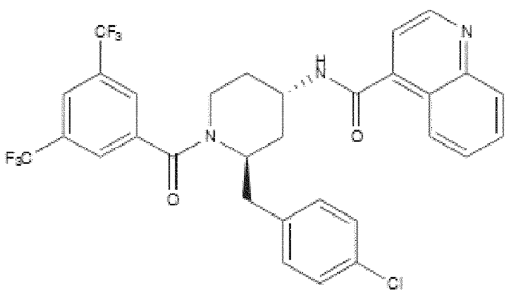
y. CP96,345 described in Lowe JA et al. 1992; 35:2591 -600, and in WO92021677, the contents of which are incorporated herein by reference in their entirety; 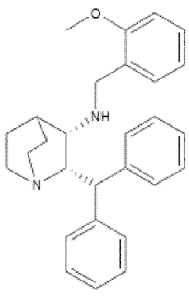
z. The following compound, described in ref. 44 and in WO92021677, the contents of which are incorporated herein by reference in their entirety; 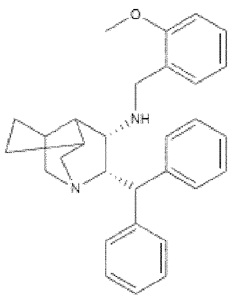
aa. CP99,994, described in reference 45, the contents of which are incorporated herein by reference in its entirety; 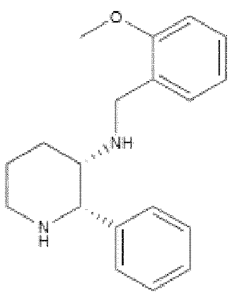
bb. CP-122,721 described in reference 46, the contents of which are incorporated herein by reference in its entirety: 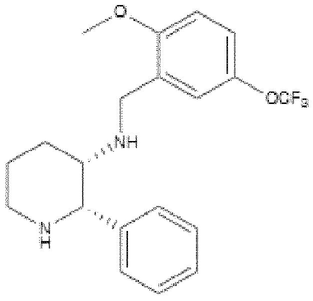
cc. CJ-17,493, described in WO9925714, the contents of which are incorporated herein by reference in its entirety: 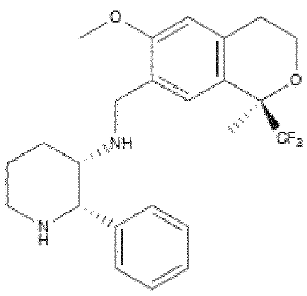
dd. Ezlopitant, CJ-1 1 ,974 described in WO1992021677 the contents of which are incorporated herein by reference in its entirety: 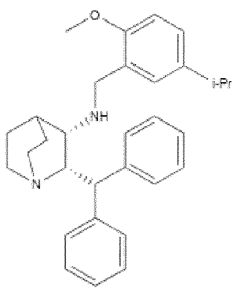
ee. Maropitant, CJ-1 1 ,972, described in WO1992021677, US 6,222,038 and US 6,255,230, the contents of which are incorporated herein by reference in their entirety: 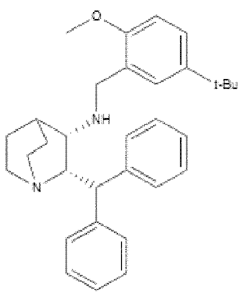
ff. RP77580 described in EP429366, the contents of which are incorporated herein reference in its entirety: 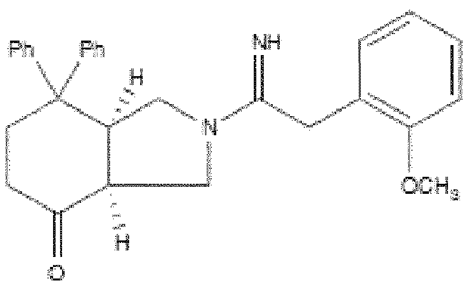
gg. Dapitant, RPR100893, described in WO9321154, the contents of which are incorporated herein by reference in its entirety: 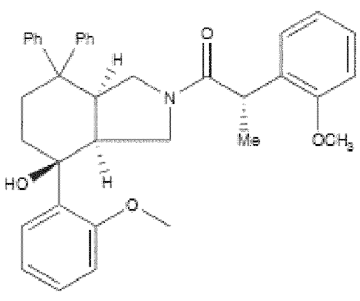
hh. The following compound, described in EP512901, the contents of which are incorporated herein by reference in its entirety: 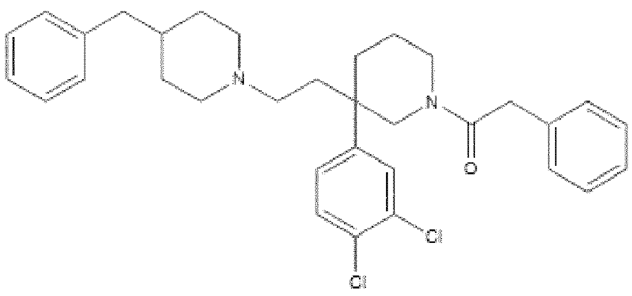
ii. Nolpitantium, SR140333 described in EP512901, the contents of which are incorporated herein by reference in its entirety: 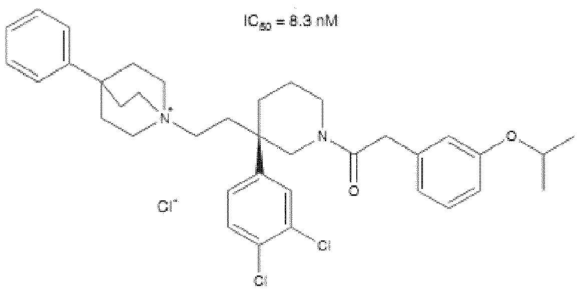
jj. The following compound, described in WO9526338, the contents of which are incorporated herein by reference in its entirety: 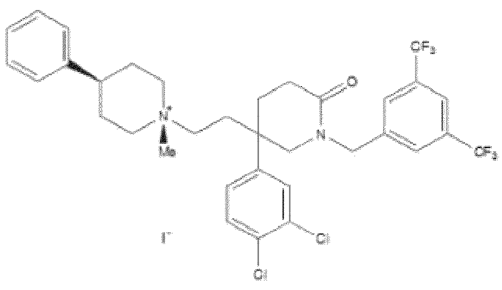
kk. SSR240600, described in WO00068292, the contents of which are incorporated herein by reference in its entirety: 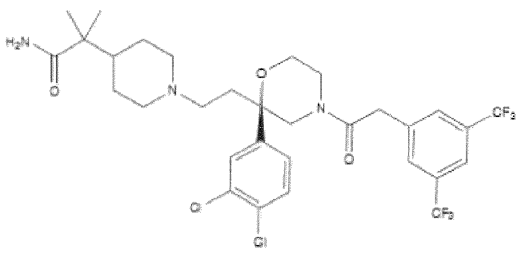
ll. SCH388714 described in WO06065654, the contents of which are incorporated herein by reference in its entirety: 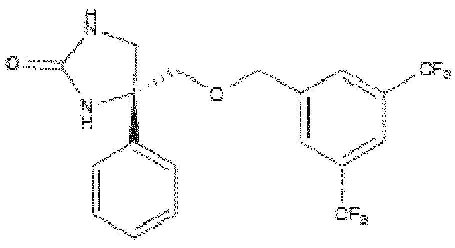
mm. The following compound described in reference 47, the contents of which are incorporated herein by reference in its entirety: 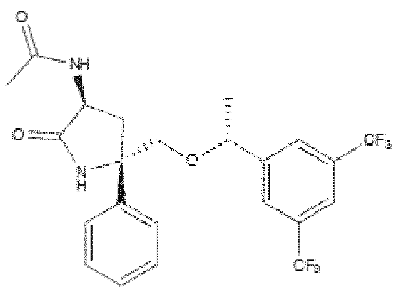
nn. Rolapitant, described in WO03051840, the contents of which are incorporated herein by reference in its entirety: 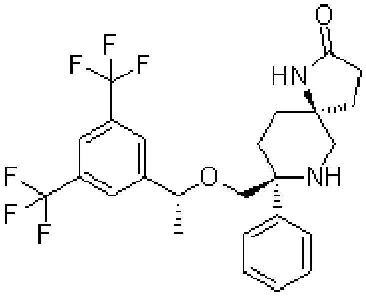
oo. The following compound, described in EP566069, the contents of which are incorporated herein by reference in its entirety: 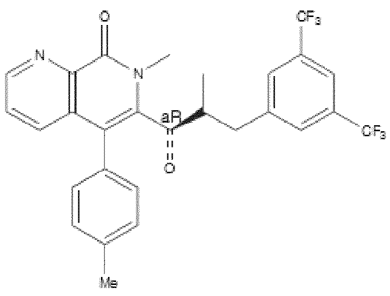
pp. TAK-637, described in JP10259184, the contents of which are incorporated herein by reference in its entirety: 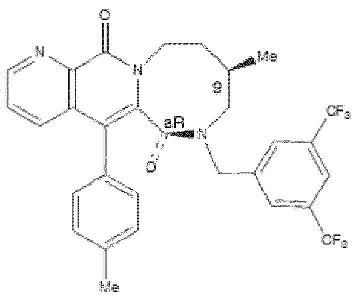
qq. The following compound described in JP2004002334, the contents of which are incorporated herein by reference in its entirety: 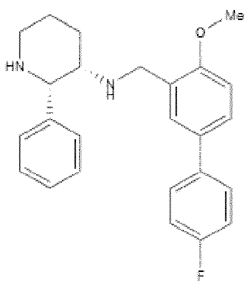
rr. The following compound described in JP2007277231 and JP2008239618, the contents of which are incorporated herein by reference in their entirety: 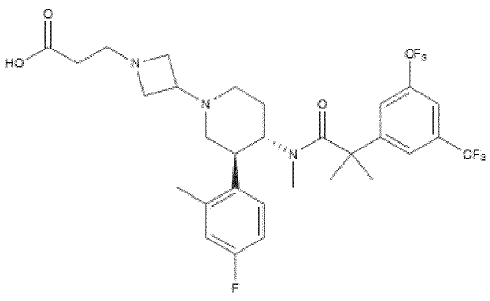
ss. The following compound described in JP2007277231 and JP2008239618, the contents of which are incorporated herein by reference in their entirety: 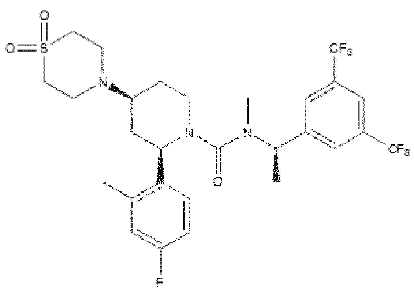
tt. The following compound described in WO9317032 and WO9511686, the contents of which are incorporated herein by reference in their entirety: 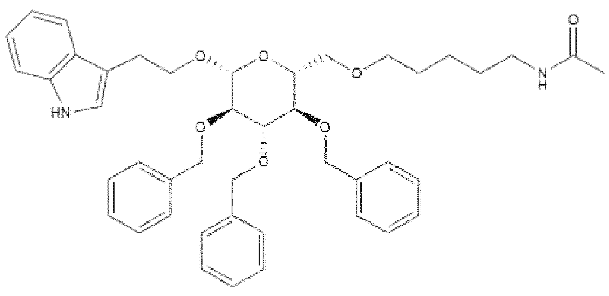
uu. The following compound described in WO9630367 and WO01025233, the contents of which are incorporated herein by reference in their entirety: 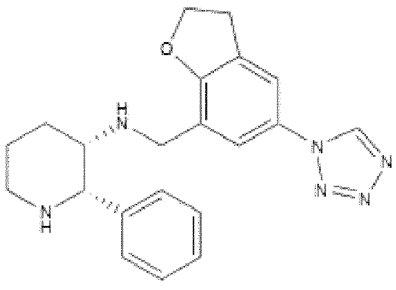
vv. HSP117 described in WO9630367 and WO01025233, the contents of which are incorporated herein by reference in their entirety: 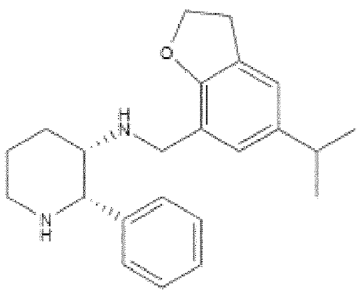
ww. The following compound, described in reference 48 and WO03062245, the contents of which are incorporated herein by reference in their entirety: 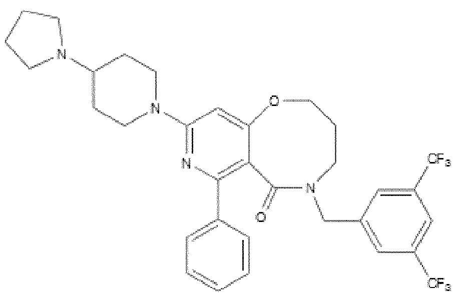
xx. The following compound, described in reference 49 and WO03062245, the contents of which are incorporated herein by reference in their entirety: 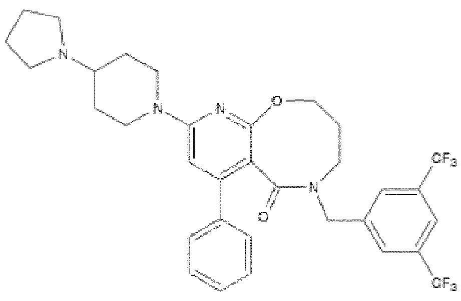
yy. The following compound, KRP-103, described in WO03062245 and WO05019225, the contents of which are incorporated herein by reference in their entirety: 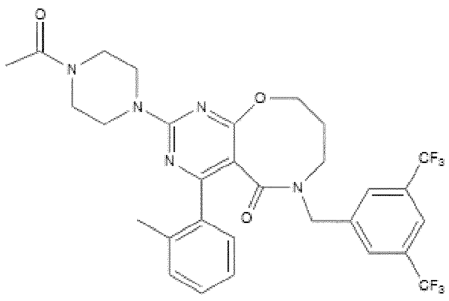
zz. The following compound described in WO06106727, the contents of which are incorporated herein by reference in its entirety: 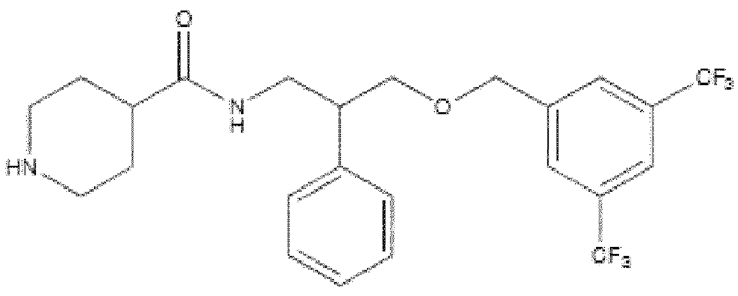
aaa. The following compound, described in WO07074491, the contents of which are incorporated herein by reference in its entirety: 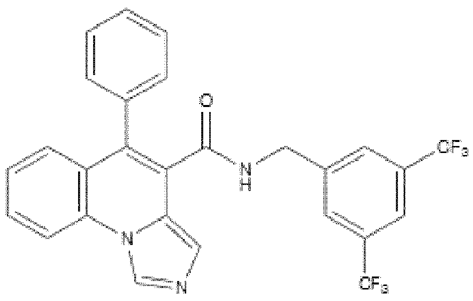
bbb. SLV317, described in US20020065276, the contents of which are incorporated herein by reference in its entirety: 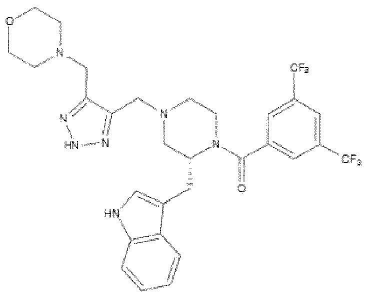
ccc. Compounds of formula I described in WO9508549, the contents of which are incorporated herein by reference in its entirety:

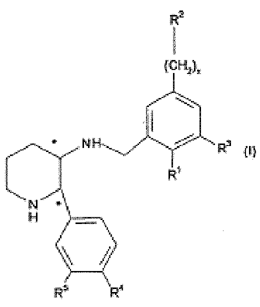

Further examples of NK-1 antagonists include but are not limited to ralopitant e varupitant. Further examples of NK-1 antagonists include but are not limited to the compounds disclosed in the following patent applications, which are incorporated herein by reference in their entirety and to which it is specifically referred to: WO9817660; US5929094, US5877191, WO00056727, WO04009573, WO00051984, WO01087838, WO02102372, WO02024629, US20050165083, WO06060346, WO06065711, WO07075528, WO06060390, WO07136570 and WO09002770.

The NK-1 antagonists according to the invention may optionally be employed in the form of a pharmaceutically acceptable salt including include salts of acidic or basic groups present in NK-1 antagonist compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate, dimeglumine and pamoate salts. Suitable base salts include, but are not limited to, aluminum, calcium, lithium, magnesium, potassium, sodium, zinc, and diethanolamine salts. Pharmaceutically acceptable salts also include hydrates.

An NK-1 Antagonist of the present invention may optionally be provided in the form of a prodrug, i.e. a precursor of a NK1 antagonist that is converted in vivo into an active or more active form ("the parent compound" or "the parent drug") by metabolic processes or other chemical breakdown event (e.g. hydrolysis). Prodrugs may conveniently be employed in compositions, methods and uses of the invention when they are more soluble than the parent compound. In some embodiments prodrugs of NK-1 antagonists contain one or more phosphate groups not possessed by the parent compound which aid water solubility. For example, a prodrug for use in the present invention is Fosaprepitant, a phosphorylated compound that rapidly converts to Aprepitant following *in vivo* administration.

### Pharmaceutical compositions and Formulations

The invention also provides a pharmaceutical composition comprising an NK-1 antagonist and a pharmaceutically acceptable vehicle for use in the treatment of ocular sensitivity and/or ocular pain. Preferably, the pharmaceutical composition of the invention is for topical ocular use and is therefore an ophthalmic composition.

The NK-1 antagonist according to the present invention can be administered by any convenient route, however the preferred route of administration is topically to the ocular surface and specially topically to the cornea. Even more preferred route is instillation into the conjunctival sac.

It is a specific object of the present invention, the use of NK-1 antagonists for the production of an ophthalmic composition to be administered topically to the eye for the therapy and/or prophylaxis of ocular sensitivity and/or ocular pain.

Accordingly, in a preferred embodiment, the invention provides a method for preventing and treating ocular sensitivity and/or ocular pain by local administration to the cornea of an ophthalmic composition comprising an NK-1 antagonist.

More generally, one preferred embodiment of the present invention is a composition formulated for topical application on a local, superficial or restricted area in the eye and/or the adnexa of the eye comprising an NK-1 antagonist optionally together with one or more pharmaceutically acceptable additives (such as diluents or carriers).

As used herein, the terms "vehicle", "diluent", "carrier" and "additive" are interchangeable.

The ophthalmic compositions of the invention may be in the form of solution, emulsion or suspension (collyrium), ointment, gel, aerosol, mist or liniment together comprising a pharmaceutically acceptable, eye tolerated and compatible with active principle ophthalmic carrier.

Also within the scope of the present invention are particular routes for ophthalmic administration for delayed release, e.g. as ocular erodible inserts or polymeric membrane "reservoir" systems to be located in the conjunctiva sac or in contact lenses.

The ophthalmic compositions of the invention may be administered topically, e.g., the composition is delivered and directly contacts the eye and/or the adnexa of the eye.

The pharmaceutical composition containing at least an NK-1 antagonist of the present invention may be prepared by any conventional technique, e.g. as described in Remington: The Science and Practice of Pharmacy 1995, edited by E. W. Martin, Mack Publishing Company, 19th edition, Easton, Pa.

In one embodiment the composition is formulated so it is a liquid, wherein the NK-1 antagonist may be in solution or in suspension. The composition may be formulated in any liquid form suitable for topical application such as eye-drops, artificial tears, eye washes, or contact lens adsorbents comprising a liquid carrier such as a cellulose ether (e.g. methylcellulose).

Preferably the liquid is an aqueous liquid. It is furthermore preferred that the liquid is sterile. Sterility may be conferred by any conventional method, for example filtration, irradiation or heating or by conducting the manufacturing process under aseptic conditions. The liquid may comprise one or more lipophile vehicles.

In one embodiment of the present invention, the composition is formulated as an ointment. Preferably one carrier in the ointment may be a petrolatum carrier.

The pharmaceutical acceptable vehicles may in general be any conventionally used pharmaceutical acceptable vehicle, which should be selected according to the specific formulation, intended administration route etc. For example, the pharmaceutical acceptable additives may be any of the additives mentioned in reference 50. Furthermore, the pharmaceutical acceptable vehicle may be any accepted additive from FDAs "inactive ingredients list", which for example is available on the internet address http://www.fda.gov/cder/drug/iig/default.htm.

At least one pharmaceutically acceptable diluents or carrier may be a buffer. For some purposes it is often desirable that the composition comprises a buffer, which is capable of buffering a solution to a pH in the range of 5 to 9, for example pH 5 to 6, pH 6 to 8 or pH 7 to 7.5.

However, in other embodiments of the invention the pharmaceutical composition may comprise no buffer at all or only micromolar amounts of buffer. The buffer may for example be selected from the group consisting of TRIS, acetate, glutamate, lactate, maleate, tartrate, phosphate, citrate, borate, carbonate, glycinate, histidine, glycine, succinate and triethanolamine buffer. Hence, the buffer may be K₂HPO₄, Na₂HPO₄ or sodium citrate.

In a preferred embodiment the buffer is a TRIS buffer. TRIS buffer is known under various other names for example tromethamine including tromethamine USP, THAM, Trizma, Trisamine, Tris amino and trometamol. The designation TRIS covers all the aforementioned designations.

The buffer may furthermore for example be selected from USP compatible buffers for parenteral use, in particular, when the pharmaceutical formulation is for parenteral use. For example, the buffer may be selected from the group consisting of monobasic acids such as acetic, benzoic, gluconic, glyceric and lactic, dibasic acids such as aconitic, adipic, ascorbic, carbonic, glutamic, malic, succinic and tartaric, polybasic acids such as citric and phosphoric and bases such as ammonia, diethanolamine, glycine, triethanolamine, and TRIS.

The compositions may contain preservatives such as thimerosal, chlorobutanol, benzalkonium chloride, or chlorhexidine, buffering agents such as phosphates, borates, carbonates and citrates, and thickening agents such as high molecular weight carboxy vinyl polymers such as the ones sold under the name of Carbopol which is a trademark of the B. F. Goodrich Chemical Company, hydroxymethylcellulose and polyvinyl alcohol, all in accordance with the prior art.

In some embodiments of the invention the pharmaceutically acceptable additives comprise a stabiliser. The stabiliser may for example be a detergent, an amino acid, a fatty acid, a polymer, a polyhydric alcohol, a metal ion, a reducing agent, a chelating agent or an antioxidant, however any other suitable stabiliser may also be used with the present invention. For example, the stabiliser may be selected from the group consisting of poloxamers, Tween-20, Tween-40, Tween-60, Tween-80, Brij, metal ions, amino acids, polyethylene glucol, Triton, and ascorbic acid.

Furthermore, the stabiliser may be selected from the group consisting of amino acids such as glycine, alanine, arginine, leucine, glutamic acid and aspartic acid, surfactants such as polysorbate 20, polysorbate 80 and poloxamer 407, fatty acids such as phosphotidyl choline ethanolamine and acethyltryptophanate, polymers such as polyethylene glycol and polyvinylpyrrolidone, polyhydric alcohol such as sorbitol, mannitol, glycerin, sucrose, glucose, propylene glycol, ethylene glycol, lactose and trehalose, antioxidants such as ascorbic acid, cysteine HCL, thioglycerol, thioglycolic acid, thiosorbitol and glutathione, reducing agents such as several thiols, chelating agents such as EDTA salts, gluthamic acid and aspartic acid.

The pharmaceutically acceptable additives may comprise one or more selected from the group consisting of isotonic salts, hypertonic salts, hypotonic salts, buffers and stabilisers.

In preferred embodiments other pharmaceutically excipients such as preservatives are present. In one embodiment said preservative is a parabene, such as but not limited to methyl parahydroxybenzoate or propyl parahydroxybenzoate.

In some embodiments of the invention the pharmaceutically acceptable additives comprise mucolytic agents (for example N-acetyl cysteine), hyaluronic acid, cyclodextrin, petroleum.

Exemplary compounds that may be incorporated in the pharmaceutical composition of the invention to facilitate and expedite transdermal delivery of topical compositions into ocular or adnexal tissues include, but are not limited to, alcohol (ethanol, propanol, and nonanol), fatty alcohol (lauryl alcohol), fatty acid (valeric acid, caproic acid and capric acid), fatty acid ester (isopropyl myristate and isopropyl n- hexanoate), alkyl ester (ethyl acetate and butyl acetate), polyol (propylene glycol, propanedione and hexanetriol), sulfoxide (dimethylsulfoxide and decylmethylsulfoxide), amide (urea, dimethylacetamide and pyrrolidone derivatives), surfactant (sodium lauryl sulfate, cetyltrimethylammonium bromide, polaxamers, spans, tweens, bile salts and lecithin), terpene (d-limonene, alpha-terpeneol, 1 ,8-cineole and menthone), and alkanone (N-heptane and N-nonane). Moreover, topically-administered compositions may comprise surface adhesion molecule modulating agents including, but not limited to, a cadherin antagonist, a selectin antagonist, and an integrin antagonist.

Also, the ophthalmic solution may contain a thickener such as hydroxymethylcellulose, hydroxyethylcellulose, hydroxypropylmethylcellulose, methylcellulose, polyvinylpyrrolidone, or the like, to improve the retention of the medicament in the conjunctival sac.

In an embodiment, the NK-1 antagonist for use according to the invention may be combined with ophthalmologically acceptable preservatives, surfactants, viscosity enhancers, penetration enhancers, buffers, sodium chloride and water to form aqueous, sterile, ophthalmic suspensions or solutions. The ophthalmic solution may further include an ophthalmologically acceptable surfactant to assist in dissolving the NK-1 antagonist. Ophthalmic solution formulations may be prepared by dissolving the NK-1 antagonist in a physiologically acceptable isotonic aqueous buffer.

In order to prepare sterile ophthalmic ointment formulations, the NK-1 antagonist may be combined with a preservative in an appropriate vehicle, such as, mineral oil, liquid lanolin, or white petrolatum. Sterile ophthalmic gel formulations may be prepared by suspending the NK-1 antagonist in a hydrophilic base prepared from the combination of, for example, carbopol-940, or the like, according to the published formulations for analogous ophthalmic preparations; preservatives and tonicity agents can be incorporated.

Preferably, the formulation of the present invention is an aqueous, non-irritating, ophthalmic composition for topical application to the eye comprising: a therapeutically effective amount of a NK1 antagonist for topical treatment of ocular pain or pharmaceutically acceptable salts thereof; a xanthine derivative being present in an amount between the amount of derivative soluble in the water of said composition and 0.05% by weight/volume of said composition which is effective to reduce the discomfort associated with the NK1 antagonist upon topical application of said composition, said xanthine derivative being selected from the group consisting of theophylline, caffeine, theobromine and mixtures thereof; an ophthalmic preservative; and a buffer, to provide an isotonic, aqueous, nonirritating ophthalmic composition.

### Drug delivery devices

In one embodiment, the invention comprises a drug-delivery device consisting of at least an NK-1 antagonist and a pharmaceutically compatible polymer. For example, the composition is incorporated into or coated onto said polymer. The composition is either chemically bound or physically entrapped by the polymer. The polymer is either hydrophobic or hydrophilic. The polymer device comprises multiple physical arrangements. Exemplary physical forms of the polymer device include, but are not limited to, a film, a scaffold, a chamber, a sphere, a microsphere, a stent, or other structure. The polymer device has internal and external surfaces. The device has one or more internal chambers. These chambers contain one or more compositions. The device contains polymers of one or more chemically-differentiable monomers. The subunits or monomers of the device polymerize in vitro or in vivo.

In a preferred embodiment, the invention comprises a device comprising a polymer and a bioactive composition incorporated into or onto said polymer, wherein said composition includes an NK-1 antagonist, and wherein said device is implanted or injected into an ocular surface tissue, an adnexal tissue in contact with an ocular surface tissue, a fluid-filled ocular or adnexal cavity, or an ocular or adnexal cavity.

Exemplary mucoadhesive polyanionic natural or semi-synthethic polymers from which the device may be formed include, but are not limited to, polygalacturonic acid, hyaluronic acid, carboxymethylamylose, carboxymethylchitin, chondroitin sulfate, heparin sulfate, and mesoglycan. In one embodiment, the device comprises a biocompatible polymer matrix that may optionally be biodegradable in whole or in part. A hydrogel is one example of a suitable polymer matrix material. Examples of materials which can form hydrogels include polylactic acid, polyglycolic acid, PLGA polymers, alginates and alginate derivatives, gelatin, collagen, agarose, natural and synthetic polysaccharides, polyamino acids such as polypeptides particularly poly(lysine), polyesters such as polyhydroxybutyrate and poly-.epsilon.-caprolactone, polyanhydrides; polyphosphazines, polyvinyl alcohols), poly(alkylene oxides) particularly poly(ethylene oxides), poly(allylamines)(PAM), poly(acrylates), modified styrene polymers such as poly(4-aminomethylstyrene), pluronic polyols, polyoxamers, poly(uronic acids), poly(vinylpyrrolidone) and copolymers of the above, including graft copolymers. In another embodiment, the scaffolds may be fabricated from a variety of synthetic polymers and naturally-occurring polymers such as, but not limited to, collagen, fibrin, hyaluronic acid, agarose, and laminin-rich gels.

One preferred material for the hydrogel is alginate or modified alginate material. Alginate molecules are comprised of (I-4)-linked β-D-mannuronic acid (M units) and a L-guluronic acid (G units) monomers which vary in proportion and sequential distribution along the polymer chain. Alginate polysaccharides are polyelectrolyte systems which have a strong affinity for divalent cations (e.g. Ca⁺², Mg⁺², Ba⁺²) and form stable hydrogels when exposed to these molecules⁵¹.

The device is administered topically, subconjunctively, or in the episcleral space, subcutaneously, or intraductally. Specifically, the device is placed on or just below the surface of an ocular tissue. Alternatively, the device is placed inside a tear duct or gland. The composition incorporated into or onto the polymer is released or diffuses from the device.

In one embodiment the composition is incorporated into or coated onto a contact lens or drug delivery device, from which one or more molecules diffuse away from the lens or device or are released in a temporally-controlled manner. In this embodiment, the contact lens composition either remains on the ocular surface, e.g. if the lens is required for vision correction, or the contact lens dissolves as a function of time simultaneously releasing the composition into closely juxtaposed tissues. Similarly, the drug delivery device is optionally biodegradable or permanent in various embodiments.

For example, the composition is incorporated into or coated onto said lens. The composition is chemically bound or physically entrapped by the contact lens polymer. Alternatively, a colour additive is chemically bound or physically entrapped by the polymer composition that is released at the same rate as the therapeutic drug composition, such that changes in the intensity of the colour additive indicate changes in the amount or dose of therapeutic drug composition remaining bound or entrapped within the polymer. Alternatively, or in addition, an ultraviolet (UV) absorber is chemically bound or physically entrapped within the contact lens polymer. The contact lens is either hydrophobic or hydrophilic.

Exemplary materials used to fabricate a hydrophobic lens with means to deliver the compositions of the invention include, but are not limited to, amefocon A, amsilfocon A, aquilafocon A, arfocon A, cabufocon A, cabufocon B, carbosilfocon A, crilfocon A, crilfocon B, dimefocon A, enflufocon A, enflofocon B, erifocon A, flurofocon A, flusilfocon A, flusilfocon B, flusilfocon C, flusilfocon D, flusilfocon E, hexafocon A, hofocon A, hybufocon A, itabisfluorofocon A, itafluorofocon A, itafocon A, itafocon B, kolfocon A, kolfocon B, kolfocon C, kolfocon D, lotifocon A, lotifocon B, lotifocon C, melafocon A, migafocon A, nefocon A, nefocon B, nefocon C, onsifocon A, oprifocon A, oxyfluflocon A, paflufocon B, paflufocon C, paflufocon D, paflufocon E, paflufocon F, pasifocon A, pasifocon B, pasifocon C, pasifocon D, pasifocon E, pemufocon A, porofocon A, porofocon B, roflufocon A, roflufocon B, roflufocon C, roflufocon D, roflufocon E, rosilfocon A, satafocon A, siflufocon A, silafocon A, sterafocon A, sulfocon A, sulfocon B, telafocon A, tisilfocon A, tolofocon A, trifocon A, unifocon A, vinafocon A, and wilofocon A. Exemplary materials used to fabricate a hydrophilic lens with means to deliver the compositions of the invention include, but are not limited to, abafilcon A, acofilcon A, acofilcon B, acquafilcon A, alofilcon A, alphafilcon A, amfilcon A, astifilcon A, atlafilcon A, balafilcon A, bisfilcon A, bufilcon A, comfilcon A, crofilcon A, cyclofilcon A, darfilcon A, deltafilcon A, deltafilcon B, dimefilcon A, droxfilcon A, elastofilcon A, epsilfilcon A, esterifilcon A, etafilcon A, focofilcon A, galyfilcon A, genfilcon A, govafilcon A, hefilcon A, hefilcon B, hefilcon C, hilafilcon A, hilafilcon B, hioxifilcon A, hioxifilcon B, hioxifilcon C, hydrofilcon A, lenefilcon A, licryfilcon A, licryfilcon B, lidofilcon A, lidofilcon B, lotrafilcon A, lotrafilcon B, mafilcon A, mesafilcon A, methafilcon B, mipafilcon A, nelfilcon A, netrafilcon A, ocufilcon A, ocufilcon B, C, ocufilcon D, ocufilcon E, ofilcon A, omafilcon A, oxyfilcon A, pentafilcon A, perfilcon A, pevafilcon A, phemfilcon A, polymacon, senofilcon A, silafilcon A, siloxyfilcon A, surfilcon A, tefilcon A, tetrafilcon A, trilfilcon A, vifilcon A, vifilcon B, and xylofilcon A.

Within the scope of the invention are compositions formulated as a gel or gel- like substance, creme or viscous emulsions. It is preferred that said compositions comprise at least one gelling component, polymer or other suitable agent to enhance the viscosity of the composition. Any gelling component known to a person skilled in the art, which has no detrimental effect on the area being treated and is applicable in the formulation of compositions and pharmaceutical compositions for topical administration to the skin, eye or mucous can be used. For example, the gelling component may be selected from the group of: acrylic acids, carbomer, carboxypolymethylene, such materials sold by B. F. Goodrich under the trademark Carbopol (e.g. Carbopol 940), polyethylene-polypropyleneglycols, such materials sold by BASF under the trademark Poloxamer (e.g. Poloxamer 188), a cellulose derivative, for example hydroxypropyl cellulose, hydroxyethyl cellulose, hydroxyethylene cellulose, methyl cellulose, carboxymethyl cellulose, alginic acid-propylene glycol ester, polyvinylpyrrolidone, veegum (magnesium aluminum silicate), Pemulen, Simulgel (such as Simulgel 600, Simulgel EG, and simulgel NS), Capigel, Colafax, plasdones and the like and mixtures thereof.

A gel or gel-like substance according to the present invention comprises for example less than 10% w/w water, for example less than 20% w/w water, for example at least 20 % w/w water, such as at least 30% w/w water, for example at least 40% w/w water, such as at least 50% w/w water, for example at least 75% w/w water, such as at least 90% w/w water, for example at least 95% w/w water. Preferably said water is deionised water.

Gel-like substances of the invention include a hydrogel, a colloidal gel formed as a dispersion in water or other aqueous medium. Thus, a hydrogel is formed upon formation of a colloid in which a dispersed phase (the colloid) has combined with a continuous phase (i.e. water) to produce a viscous jellylike product; for example, coagulated silicic acid. A hydrogel is a three-dimensional network of hydrophilic polymer chains that are crosslinked through either chemical or physical bonding. Because of the hydrophilic nature of the polymer chains, hydrogels absorb water and swell. The swelling process is the same as the dissolution of non-crosslinked hydrophilic polymers. By definition, water constitutes at least 10% of the total weight (or volume) of a hydrogel.

Examples of hydrogels include synthetic polymers such as polyhydroxy ethyl methacrylate, and chemically or physically crosslinked polyvinyl alcohol, polyacrylamide, poly(N-vinyl pyrrolidone), polyethylene oxide, and hydrolyzed polyacrylonithle. Examples of hydrogels which are organic polymers include covalent or ionically crosslinked polysacchahde-based hydrogels such as the polyvalent metal salts of alginate, pectin, carboxymethyl cellulose, heparin, hyaluronate and hydrogels from chitin, chitosan, pullulan, gellan and xanthan. The particular hydrogels used in our experiment were a cellulose compound (i.e. hydroxypropylmethylcellulose [HPMC]) and a high molecular weight hyaluronic acid (HA).

Hyaluronic acid is a polysaccharide made by various body tissues. U.S. patent 5,166,331 discusses purification of different fractions of hyaluronic acid for use as a substitute for intraocular fluids and as a topical ophthalmic drug carrier. Other U.S. patent applications which discuss ocular uses of hyaluronic acid include serial numbers 11/859,627; 11/952,927; 10/966,764; 11/741,366; and 11/039,192 Formulations of macromolecules for intraocular use are known, See eg U.S. patent applications serial numbers 11/370,301; 11/364,687; 60/721,600; 11/116,698 and 60/567,423; 11/695,527. Use of various active agents is a high viscosity hyaluronic acid is known. See eg U.S. patent applications serial numbers 10/966,764; 11/091 ,977; 11/354,415; 60/519,237; 60/530,062, and; 11/695,527.

Sustained release formulations as described in WO2010048086 are within the scope if the invention.

### Concentration of active ingredient and therapeutic regimen

Compositions and pharmaceutical compositions according to the present invention, comprise at least one NK-1 antagonist as an active ingredient. The concentration of NK-1 antagonist in said compositions may vary according to the type of administration they are formulated for. The compositions may comprise 0.1 ng/ml to 10 mg/ml, preferably 100 ng/ml to 10 mg/ml, such as 100 g/ml to 10 mg/ml, preferably 1 mg/ml to 10 mg/ml NK-1 antagonist.

In a preferred embodiment, pharmaceutical compositions according to the present invention comprise at least 10 mg/ml of active ingredient. In another preferred embodiment, pharmaceutical compositions according to the present invention comprise at least 50 mg/ml of active ingredient.

Accordingly, the total dose per day of active principle may comprise 10 ng to 100 mg, preferably 100 ng to 10 mg, preferably 10µg to 10mg, preferably 200µg to 1 mg, preferably 200µg, of NK-1 antagonist. In a preferred embodiment, the total dose per day of active principle is of at least 50 mg, preferably of at least 60 mg.

The compositions may comprise 0,01 to 50 % (weight/volume) of NK-1 Antagonist, preferably 0,05 to 5 % (weight/volume), more preferably 0,05 to 1 wt% (weight/volume), or most preferably 0,1 to 2% (weight/volume) of the NK-1 Antagonist, for example the composition may comprise 0,05% (weight/volume), 0,075% (weight/volume), 0,1 % (weight/volume), 1%, (weight/volume), 2%(weight/volume, 40% (weight/volume), 5%(weight/volume), of NK-1 antagonist.

According to the present invention "a therapeutically effective amount" of the composition refers to the amount necessary to induce the desired biological effect on the subject in need of treatment.

The compositions and pharmaceutical compositions according to the present invention may be administered once or several times per day, for example they may be administered in the range of 2 to 10 times a day, such as e.g. 2 to 8 times, for example 2 to 6 times, such as 2 to 4 times, such as 2 to 3 times a day. Preferably, the NK-1 antagonists and the pharmaceutical compositions according to the present invention are administered six times a day.

The compositions according to the present invention may be administrated to the subject for a period of treatment of one or more than one week such as two weeks, three weeks, four weeks, five weeks, six weeks, seven weeks, eight weeks or more than eight weeks. The treatment may be repeated on subjects who relapse.

Advantageously, the NK-1 antagonists and the pharmaceutical compositions according to the present invention may be administered to the subject only once using the above-defined dosages.

A further aspect of the present invention relates to a method of treating or ameliorating a medical condition of the eye characterized by the presence of ocular sensitivity and/or ocular pain comprising administration to an animal subject including a human being in need thereof an effective dosage of a composition or a pharmaceutical composition as defined herein above.

### Combination therapy

In one embodiment the treatment or prevention of ocular sensitivity and/or ocular pain consists of the use of an NK-1 antagonist as sole pharmaceutically active agent.

However, in certain embodiments the invention further encompasses the administration of an NK-1 antagonist concurrently with one or more further therapeutically active agents that are administered to the same patient, each active agent being administered according to a regimen suitable for that medicament. This encompasses pre-treatment, simultaneous treatment, sequential treatment, and alternating regimens.

The one or more therapeutically active agents may be administered by the same route as the NK-1 antagonist or by a different route (or by one or more different routes).

At least one of the one or more further therapeutically active agents may, for example, administered topically to the eye.

Examples of such active agents include but are not limited to antivirals, antibacterial agents (such as antibiotics), analgesics, antagonists of inflammatory cytokines, corticosteroids, non-steroidal anti-inflammatory agents, immunosuppressants, anti- fungal agents and anesthetics. Preferably, the one or more further therapeutically active agent may be an agent that is useful in the prevention and/or treatment of ocular sensitivity and ocular pain, such as an anesthetic agent, a non-steroidal anti-inflammatory agent or an analgesic. Still preferably, the one or more further therapeutically active agent may be an agent that is useful in the prevention and/or treatment of the disease or condition that causes the ocular sensitivity and/or ocular pain or an agent that is used following surgery to the eye. In one specific embodiment, the invention encompasses a method of treating or preventing ocular sensitivity and/or ocular pain by administering an NK-1 antagonist concurrently with an antibiotic agent.

In one specific embodiment there is provided a pharmaceutical composition suitable for topical administration to the eye comprising an NK-1 antagonist and an antibiotic agent.

Typically, such a composition will comprise one or more diluents or carriers which are pharmaceutically acceptable for topical administration to the eye.

In another embodiment, the one or more further therapeutically active agents are selected from VEGF inhibitors, II,1-Rinhibitors, immunosuppressants and TNF inhibitors.

In one embodiment of the invention, one of the one or more further therapeutically active agents is an antibiotic such as amikacin, gentamicin, kanamycin, neomycin, netilmicin, streptomycin, tobramycin, teicoplanin, vancomycin, azithromycin, clarithromycin, clarithromycin, dirithromycin, erythromycin, roxithromycin, troleandomycin, amoxicillin, ampicillin, azlocillin, carbenicillin, clozacillin, dicloxacillin, flucozacillin, mezlocillin, nafcillin, penicillin, piperacillin, ticarcillin, bacitracin, colistin, polymyxin B, ciprofloxacin, enoxacin, gatifloxacin, levofloxacin, lomefloxacin, moxifloxacin, norfloxacin, oflazacin, trovafloxacin, mafenide, sulfacetamide, sulfamethizole, sulfasalazine, sulfisoxazole, tetracycline, trimethoprim, cotrimoxazole, demeclocycline, soxycycline, minocycline, doxycycline, oxytetracycline or tetracycline.

In a further embodiment of the invention, one of the one or more further therapeutically active agents is an immunosuppressive agent such as cyclosporin A.

In a further embodiment of the invention, one of the one or more further therapeutically active agents is an antagonist of inflammatory cytokines such as antagonist of tumor necrosis factor alpha (TNFα). Exemplary functional blockers of TNFα include, but are not limited to, recombinant and/or soluble TNFα receptors, monoclonal antibodies, and small molecule antagonists and/or inverse agonists. One or more commercially-available TNF-a blocking agents are reformulated for topical administration in this embodiment. Exemplary commercial TNF-a blocking agents used for reformulation include, but are not limited to, etanerept/Embrel, infliximab/Remicade, and adalimumab/Humira.

Alternatively, one of the one or more further therapeutically active agents is an antagonist of an inflammatory cytokine selected from IL-I, IL-2, IL-4, IL-5, IL-6, IL-8, IL-12, IL-17, IL-18 and IL-23.

In a further embodiment of the invention, one of the one or more further therapeutically active agents is an antagonist of one or more member(s) of the vascular epithelial growth factor (VEGF) family. Exemplary members include, but are not limited to, VEGF-A, VEGF- C, VEGFR-2, and VEGFR-3. Anti-VEGF agents which inhibit either VEGF itself or the VEGF receptor present in the eye in order to thereby prevent angiogenesis, include but are not limited to monoclonal antibodies such as ranibizumab (LUCENTIS^{®}; rhuFab V2) and bevacizumab (AVASTIN^{®}; rhuMab-VEGF), nucleic acids (aptamers such as MACUGEN^{®}, (pegaptanib) a PEGylated RNA aptamer, and siRNAs directed to VEGF RNA). Bevacizumab is a full-length anti- VEGF antibody approved for use in metastatic colon cancer. Ranibizumab is a humanized anti-VEGF monoclonal antibody fragment that inhibits all isotypes of VEGF and pegaptanib is a VEGF-neutralizing aptamer that specifically inhibits one isoform of VEGF (VEGF-165).

Further examples include antibody fragments (e.g. Ranibizumab), small interfering RNA's decreasing expression of VEGFR or VEGF ligand, post-VEGFR blockade with tyrosine kinase inhibitors, Small molecule RTK inhibitors targeting VEGF receptors including PTK787 can also be used.

In a further embodiment of the invention, one of the one or more further therapeutically active agents is an antagonist of interferon-gamma.

In a further embodiment of the invention, one of the one or more further therapeutically active agents is an antagonist of one or more chemokines and their receptors. Exemplary chemokines and receptors that may be antagonized by a further active agent include chemokine (C-C motif) receptor 1 (CCRI), chemokine (C-C motif) receptor 2 (CCR2), chemokine (C-C motif) receptor 5 (CCR5), chemokine (C-C motif) receptor 7 (CCR7), and chemokine (C-X-C motif) receptor 3 (CXCR3).

The invention will be now illustrated by means of non-limiting examples referring to the following figures.

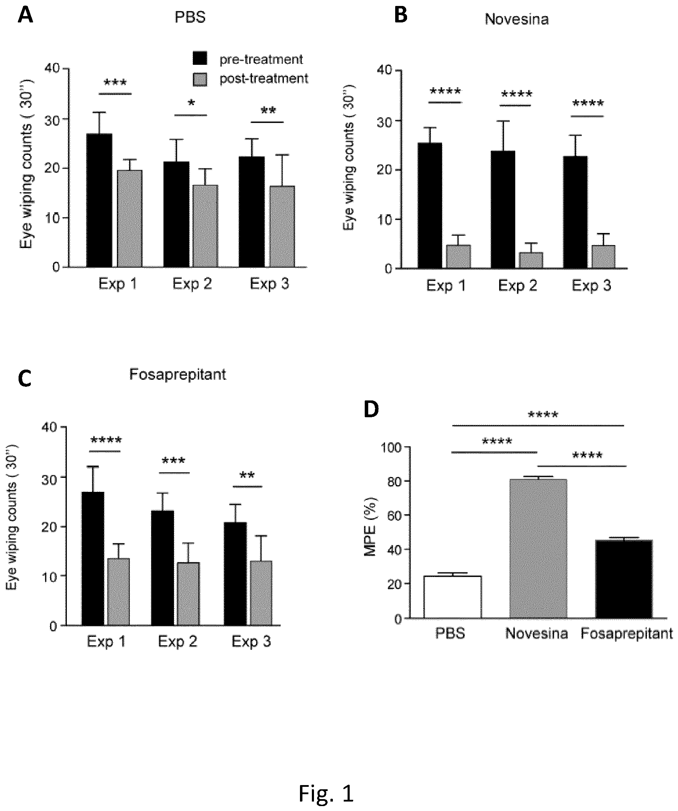

**Figures 1A-1C****.** Topical Fosaprepitant 50 mg/ml administered once significantly reduces corneal pain measured as Eye wiping counts 30 minutes after Fosaprepitant administration as compared with placebo. Novesina (Oxybuprocaine 4mg/ml), a known topical anesthetic significantly reduces corneal pain as compared with placebo and Fosaprepitant. **Figure 1D****.** Novesina and Fosaprepitant (10 microl instilled in the conj. sac) significantly reduce corneal pain with respect to placebo (measured as maximum possible effect, MPE). Novesina, a known topical anesthetic, reduces corneal pain significantly more than Fosaprepitant.

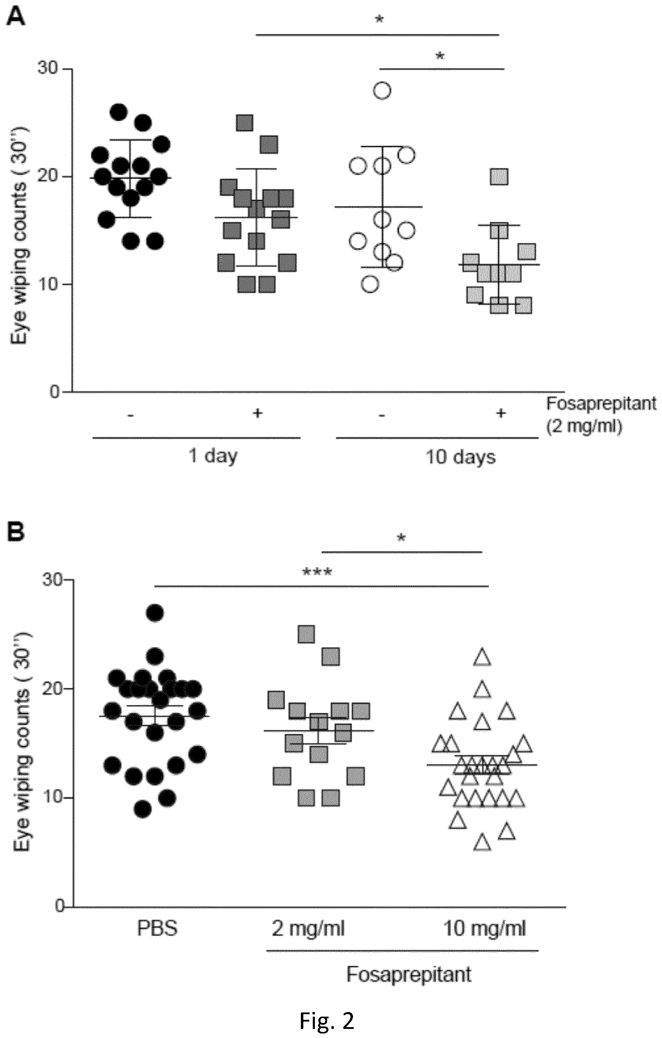

**Figure 2****.** Long-term Fosaprepitant administration (2 mg/ml) and single topical Fosaprepitant administration (10 mg/ml) reduce corneal nociception. **A)** Eye-wiping counts after Fosaprepitant administration 2 mg/ml once (dark grey) *vs* 6 times a day for 10 days (light gray) vs controls (black and white respectively). **B)** Single topical Fosaprepitant administration 2 mg/ml (gray squares) vs 10 mg/ml (white triangles) vs control (black circles). Graphs represent mean values ± SEM; Statistical analysis by unpaired Student's t test (*p<0.05, ***p<0.001).

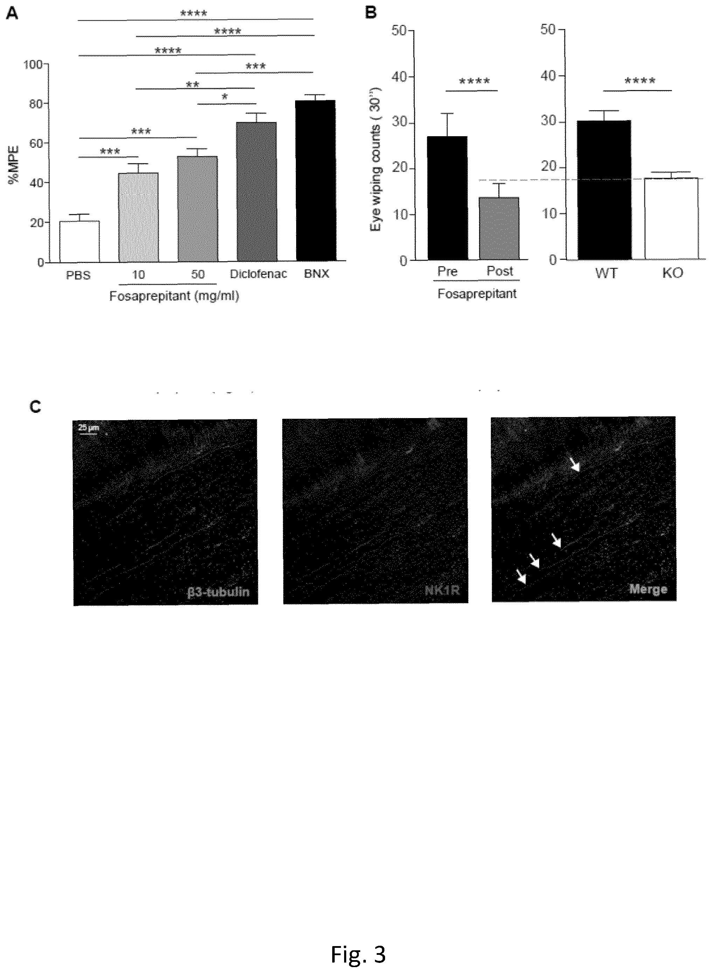

**Figure 3****.** Fosaprepitant causes a milder reduction of cornea nociception compared to Oxybuprocaine, reaching SP-KO mouse corneal nociception levels. **A)** MPE% (maximum possible effect) of PBS, Fosaprepitant (10 and 50 mg/ml), Diclofenac and Oxybuprocaine (BNX), in reducing mouse cornea nociception. **B)** WT animals nociception before (black) and after (grey) Fosaprepitant administration (10 mg/ml) compared to SP-KO mice nociception (right panel, white bar). The red dashed line compares WT after Fosaprepitant to SP-KO nociception. **C)** β3-tubulin (left image) and NK1R (central image) co-staining showed partial co-localization (right image, white arrows), in mouse corneal whole mounts. Graphs represent mean values ± SEM; Statistical analysis by unpaired Student's t test (*p<0.05, **p<0.01, ***p<0.001, ****p<0.0001).

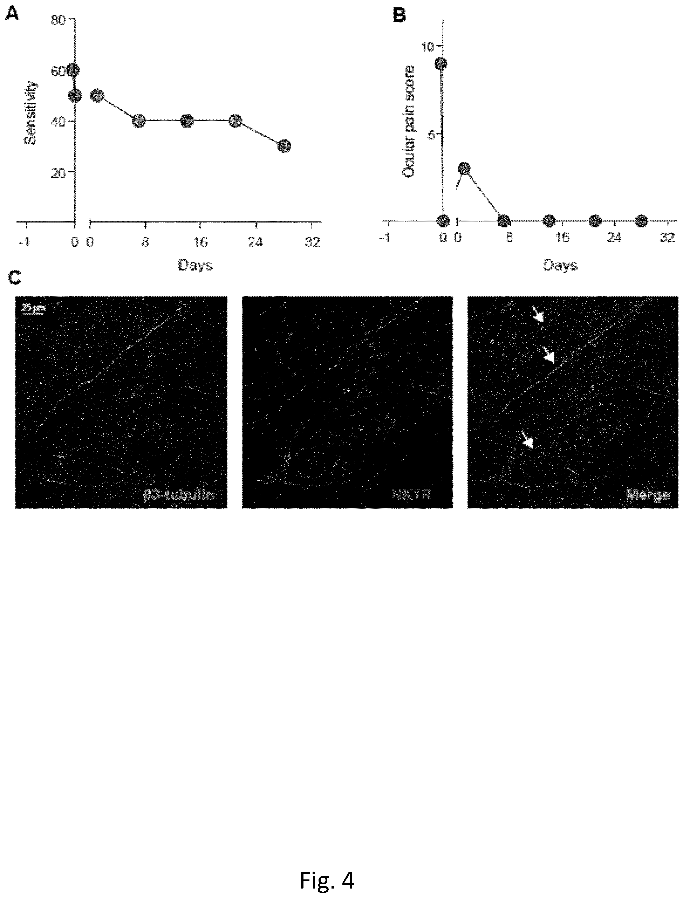

**Figure** 4. Topical instillation of Fosaprepitant reduces patient ocular sensitivity and pain. Measurement of corneal sensitivity **(A)** and ocular pain score **(B)** on a patient treated 30 days with Fosaprepitant 10 mg/ml. **C)** β3-tubulin (left image) and NK1R (central image) co-staining showed partial co-localization (right image, white arrows), in human corneal whole mounts.

### DETAILED DESCRIPTION OF THE INVENTION

### Materials and methods

### Mice

8 week-old C57BL6/N (Charles-River, Italy) and B6.Cg-Tac1^{tm1Bbm}/J (SP-KO mouse, Jackson) male mice were used in all experiments. Carbon dioxide inhalation and subsequent cervical dislocation were applied to euthanize the animals. All experimental protocols were approved by the Animal Care and Use Committee of the IRCCS San Raffaele Scientific Institute, in accordance with the ARVO Statement for the Use of Animals in Ophthalmic and Vision Research.

### Measurement of cornea sensitivity - Eye-wiping test

Mice 8-week-old C57BL6/N (Charles-River, Italy) and B6.Cg-Tac1^{tm1Bbm}/J (Jackson) corneal sensitivity was measured with the eye wiping test³⁶. Briefly, animals were placed individually in an empty cage for 5 min to get acclimatized; one drop (10 µl) of NaCl 5M was put into the right eye of the animal and eye wipings with the ipsilateral forepaw were counted for 30 s. Subsequently, a drop (10 µl) of Fosaprepitant (2, 10 and 50 mg/mL, powder was weighted and dissolved in PBS to reach the desired final concentration, Ivemend, Merck Sharp & Dohme B.V), PBS, Diclofenac (0.1%, Dicloftil, Farmingea) or Oxybuprocaine chloride (4mg/ml, Novesina, Laboratoire Thea) was put on the same eye and left for 3 min. 5 minutes after the treatment, the eye was stimulated a second time with NaCl 5M and wipings were counted for 30 s. Three experiments were performed, each with 8 mice per group. In a sub-group of experiments, cornea sensitivity was tested 24 hours after 10 days of topical treatment (10 µl) with 2 mg/mL Fosaprepitant (6 times/day).

Anti-nociceptive effect was calculated as maximal possible effect (%MPE or MPE%) for each mouse according to the following formula: % MPE = 100 X (post-treatment wipe count - pre-treatment wipe count) / (0 - pre-treatment wipe count).

### Immunofluorescence

To evaluate the expression of NK1 receptor on corneal nerves, whole-mount mice cornea were immunostained as previously described³⁷. Briefly, freshly excised corneas were washed in PBS and fixed in acetone at 4°C for 15 minutes. Nonspecific staining was blocked with 2% BSA, 5% normal donkey serum following immunostaining with rabbit anti-β3 tubulin (Millipore, Burlington, Massachusetts) and goat anti-NK1R (Santa Cruz, Dallas, Texas) primary antibodies 16 hours at 4°C. After washing with PBS, cornea were incubated with Alexa 488 donkey anti-rabbit and Alexa 555 donkey anti-goat secondary antibodies (Invitrogen, Carlsbad, California) 2 hours at RT and mounted with Vector Shield mounting medium (Vector Laboratories, Burlingame, CA). For human co-localization experiments, keratoconus affected patients (n=5, 3 males, 2 females, age 43.4 ± 7.7 years) planning to undergo corneal transplant were selected, after obtaining consent, by the Cornea and Ocular Surface Unit at the San Raffaele Scientific Institute. At the time of corneal transplant, fresh corneal buttons obtained from these patients were collected and processed as the murine counterpart for β3 tubulin and NK1R double staining. Mouse anti-β3 tubulin (San Diego, California) and anti-NK1R (Santa Cruz, Dallas, Texas) primary antibodies were used.

For both human and mouse, cornea images (40x, 5 µm z-stack) were taken with confocal microscope (TCS SP5; Leica Microsystems) and co-localization was assessed with ImageJ.

### Patient treatment

One 84-year-old female patient affected with Stage II (Foster) ocular cicatritial pemphigoid
and intractable ocular pain was recruited on a compassionate trial of Fosaprepitant, upon administration and acceptance of informed consent, after consultation with the San Raffaele Hospital Ethics Committee. The patient reported the pain to be located on the ocular surface of both eyes and rated it 9/10 on the right eye and 2/10 on the left eye. Visual acuity was light perception in both eyes and the left eye presented a descemetocele. In the right eye the cornea presented diffuse punctate keratopathy, and conjunctival scarring. All the medications in use at time of enrollment were maintained. These included topical medications: tacrolimus 0.02% 3 times a day, dexamethasone phosphate twice a day, Timolol-brinzolamide drops twice a day, lubricants 6 times a day. In addition, the patient was taking systemic prednisone (25 mg); other systemic immunosuppressants had been previously tried to control the disease but had been suspended due to inefficacy or intolerance. In an unsuccessful attempt to control ocular pain, the prednisone dosage was raised up to 25 mg per day and the patient had been started 3 months before with opioid analgesic Tapentadol 50 mg bid and paracetamol 1000 mg tid. Only the right eye was treated. In this study, the patient received treatment with ocular Fosaprepitant 10 mg/mL (50 µl) 6 times/day for 30 days.

### Sensitivity (Cochet-Bonnet) test and pain score.

To test corneal sensitivity, a Cochet-Bonnet aesthesiometer (60 mm in length, 0.12 mm diameter) was used. The head of the subject was placed on the chinrest of the slit lamp, and the Cochet-Bonnet filament was neared the volunteer's eye with a perpendicular angle. Different filament lengths were tried (starting from 60 mm, 5 mm intervals) until response, and noted. In order to quantify subjective ocular pain, the treated patient was asked to rate the pain felt in the treated eye on a scale from 0 (no pain) to 10 (worst possible pain) before and after the treatment³⁸.

### Statistics

Unpaired t-test was used to evaluate the differences in nociception after different treatments. A p value <0.05 was considered to be statistically significant. The statistical software GraphPad Prism 5.0 (GraphPad Software, La Jolla, CA) was used for all analyses. All data were expressed as mean ± standard error of the means (SEM). All methods were performed in accordance with the relevant guidelines and regulations.

### Examples

### Example 1: The effect of topically applied phosphate buffered saline, novesine (oxybuprocaine) and Fosaprepitant on corneal nociception.

Topical instillation of Novesine, induced the maximum reduction in the number of wipe counts (i.e. reduction in corneal nociception), followed by Fosaprepitant and PBS (Fig 1A, B, C). MPE measured following topical PBS application was 25, 20.5, 27.2 (SD: 15.3, 13, 21.1) in the first, second and third experiment respectively (Fig. 1). MPE following oxybuprocaine chloride 4mg/ml application was 80.8, 84.1, and 78.2 (SD: 9.7, 13.9, 10.9) in the three different experiments (Fig. 1). Following application of Fosaprepitant 50mg/ml, MPE was 48.4, 43.2, and 44.2 respectively (SD: 14.6, 24.9, 16.6) (Fig. 1).

Overall, topical application of Oxybuprocaine chloridrate significantly reduced corneal sensitivity (MPE increase of 56.92 points; p<0.0001). Topical Fosaprepitant also significantly reduced corneal sensitivity (MPE increase of 21.03 points; p<0.0001). Finally, when comparing topical Fosaprepitant with Oxybuprocaine, it was also observed that Oxybuprocaine was significantly more anesthetic than Fosaprepitant (35.89 MPE points higher; p<0.0001), Fig. 1D.

### Example 2: Low-dose long-term Fosaprepitant administration reduces corneal nociception

In the present invention, it was found that topical application of Fosaprepitant 2 mg/ml one time did not significantly reduce cornea sensitivity (Figure 2A). On the contrary, topical administration of the same concentration of drug 6 times a day for 10 days significantly reduced cornea sensitivity (-31.4%, p=0.0195 compared to 10 days PBS instillation, - 27,1%, p=0.0174 single shot vs 10 days Fosaprepitant treatment, Figure 2A).

### Example 3: High-dose single topical Fosaprepitant administration induces corneal analgesia

In a second group of experiments, the comparison of a single administration of two different concentrations of Fosaprepitant (2 mg/ml, 10 mg/ml) showed a significant reduction of nociception in the group of mice treated with the highest dose of drug (-28,1%, p=0.029 10 mg/ml vs PBS, -23,1%, p=0.0005 10 mg/ml vs 2 mg/ml, Figure 2B).

### Example 4: Fosaprepitant causes a milder reduction of cornea nociception compared to Diclofenac and Oxybuprocaine, reaching SP-KO mouse levels of corneal nociception

The maximum possible analgesic effect (MPE) measured following topical application of Fosaprepitant 10 and 50 mg/ml was significantly higher than the MPE of control treatment with PBS (respectively 44.52 and 52.62 *vs* 20.59 p=0.0006 and p=0.0001, Figure 3A). The %MPE difference between the two Fosaprepitant concentrations was however not significant. Topical application of Diclofenac 0.1% or Oxybuprocaine 4mg/ml resulted to be the most effective treatments in reducing corneal nociception (%MPE 69.66 and 80.59, p=0.0029 and p<0.0001 vs Fosaprepitant 10mg/ml respectively, Figure 3A).

Last, we quantified corneal sensitivity in tachykinin-KO mice lacking Substance P, the main ligand of Neurokinin 1 receptor. We observed that KO mice showed a reduced corneal sensitivity compared to WT mice (-41.7%, p<0.0001, Figure 3B). This sensitivity reduction resulted to be comparable to the one seen in the group of mice treated with a single administration of Fosaprepitant 10 mg/ml (-47,9, p<0.0001 Figure 3B).

### Example 5: Corneal sub-basal nerves express NK1 receptor

β3-tubulin and anti-NK1R double staining of WT whole-mount corneas showed a co-localization of the two proteins, indicating that corneal sub-basal nerves express NK1R, the receptor for substance P (Figure 3C, white arrows).

### Example 6: Daily instillation of Fosaprepitant reduces patient ocular sensitivity and pain

Finally, we treated an 84-year-old patient affected with severe ocular pain due to ocular cicatritial pemphigoid, with topical Fosaprepitant 10 mg/ml, 6 times a day for 30 days. 2 hours after the first instillation corneal pain dropped from 9/10 to 0/10, Cochet Bonnet aesthesiometry from 60 to 50 mm. After one month of treatment, pain score remained stable with a grade of 0/10 (Fig 4B), Cochet Bonnet aesthesiometry was reduced by 50% (Figure 4A). A follow-up visit performed one month after study exit showed that corneal sensitivity was back to normal (60mm), and pain score was 1/10 and not constant. As in mice, β3-tubulin and NK1R partially co-localized in a whole-mount human cornea (Figure 4C).

Ocular pain represents a significant medical problem, and an area of current unmet medical need. Topical anesthetics are highly effective, but they can only be administered for a limited period of time and are associated with significant side effects such as toxic keratopathy, corneal melting and perforation, which make their safety profile unacceptable for many authors¹⁹⁻²². Moreover, their use does not seem to be beneficial in pain control therapies of corneal abrasions²³, a common cause of ocular pain. In this vein, a recent meta-analysis did not find any significant improvement in symptoms and pain in patients treated with topical anesthetics versus placebo²⁴.

Topical NSAIDs have been also proposed for the treatment of corneal pain. Although some analgesic efficacy was demonstrated, the limited sample size of the studies made it difficult to draw definitive conclusions²³. NSAIDs are generally less effective than topical anesthetics, and a recent Cochrane meta-analysis failed to provide strong evidence supporting the use of NSAIDs in corneal abrasions, a common cause of ocular pain²⁵. From the safety perspective, long-term NSAIDs therapies can also be detrimental, by impairing corneal wound healing and inducing corneal melting and perforation²⁶⁻²⁹.

Systemic analgesics can successfully control ocular pain, although their use is associated with significant side effects (e.g: reduced alertness, hallucinations, gastro-intestinal, liver and kidney toxicity)³⁰. In addition, when pain is limited to the ocular surface, there is limited rationale for using systemic pain control, if topical alternatives are available.

In the present invention it is shown that topical inhibition of NK1R by means of a selective NK1 inhibitor, Fosaprepitant, is effective in reducing pain in an animal model of corneal pain. It was observed that the lower effective concentration in reducing corneal pain with a single administration was 10 mg/ml. The lower analgesic efficacy exhibited by Fosaprepitant in the present animal studies does not necessarily translate into reduced clinical efficacy, at least in inflamed eyes, since the treated patient reported dramatic pain reduction. Interestingly, topical application of Fosaprepitant 10 mg/ml 6 times a day did not show obvious signs of toxicity to the cornea in animal models of severe ocular surface inflammation¹⁷.

In addition, reduced corneal pain and sensitivity in Substance P knock-out mice is reported. Since Substance P is the primary ligand of the Neurokinin 1 receptor, it is concluded that either pharmacological inhibition of NK1R or congenital absence of its ligand SP is able to reduce corneal sensitivity and pain. Importantly, a significant reduction in corneal nerve density between normal and KO mice³¹ was not observe, hence suggesting that absence of SP is not detrimental to the maintenance of normal nerve morphology in the cornea, at least in young mice.

The effects on pain of NK1 antagonists administered systemically have been extensively debated as some clinical trials obtained beneficial effects^{32,33} while, in others, no effect was observed in non-ocular conditions^{34,35}. Still, the fact that in the present invention, ocular analgesic effects were observed minutes after administration suggests that Fosaprepitant may be acting through a local mechanism, as therapeutically active systemic concentration would require a longer time, and are expected to be minimal anyway. Then, such local effect could be mediated by NK1 receptors located directly on corneal nerves. Indeed, immunostaining of human and mouse corneas suggests corneal nerves express NK1R.

Finally, no gross signs of ocular surface toxicity associated with this treatment in mice¹⁷, and in the human subject were observed.

The present findings that pre-treatment with a NK1 inhibitor (Fosaprepitant) reduces ocular trigeminal pain makes its use before surgery (refractive surgery, corneal crosslinking, cataract surgery, and, in general, corneal surgery) extremely attractive. On a broader perspective, since ocular pain affects millions worldwide, topical NK1 inhibitor treatment such as Fosaprepitant represent a promising therapeutic tool.

### BIBLIOGRAPHIC REFERENCES

1. Belmonte C, Nichols JJ, Cox SM, et al. Ocul Surf. Jul 2017;15(3):404-437.
2. Bowen RC, Koeppel JN, Christensen CD, et al. J Neuroophthalmol. Jan 12 2018.
3. Lisch W. Klin Monbl Augenheilkd. Jun 2013;230(6):582-586.
4. Borsook D, Rosenthal P. Pain. Oct 2011;152(10):2427-2431.
5. Kaido M, et al., Invest Ophthalmol Vis Sci. Mar 2016;57(3):914-919.
6. Ghanem VC, Ghanem RC, de Oliveira R. Cornea. Jan 2013;32(1):20-24.
7. Wang D, Chen G, Tang L, Li Q. Eye Sci. Sep 2014;29(3):155-159.
8. Garcia R, et al., Clin J Pain. May 2016;32(5):450-458.
9. Walters T, et al., J Cataract Refract Surg. Oct 2015;41(10):2049-2059.
10. McVeigh K, Vahdani K, Tavassoli S, Tole D. BMJ. Sep 14 2017;358:j3614.
11. Yawn BP, et al., Mayo Clin Proc. Jun 2013;88(6):562-570.
12. Rosenthal P, Borsook D. Br J Ophthalmol. Jan 2016;100(1):128-134.
13. Goyal S, Hamrah P. Semin Ophthalmol. 2016;31(1-2):59-70.
14. Navari RM. Expert Opin Investig Drugs. Dec 2007;16(12):1977-1985.
15. Aapro M, et al., Oncologist. Apr 2015;20(4):450-458.
16. Saito H, Yoshizawa H, Yoshimori K, et al. Ann Oncol. Apr 2013;24(4):1067-1073.
17. Bignami F, et al., Acta Ophthalmol. Nov 2017;95(7):e641-e648.
18. Muller and Tervo (2003) Exp Eye Res, 76, 521 -542
19. Wu H, Hu Y, Shi XR, et al. Exp Ther Med. Jul 2016; 12(1):343-346.
20. Chen HT, Chen KH, Hsu WM. Cornea. Jul 2004;23(5):527-529.
21. Rao SK, et al., J Cataract Refract Surg. Aug 2007;33(8):1482-1484.
22. Sugar A. J Cataract Refract Surg. Nov 1998;24(11):1535-1537.
23. Thiel B, Sarau A, Ng D. Cureus. Mar 27 2017;9(3):e1121.
24. Puls HA, et al., J Emerg Med. Nov 2015;49(5):816-824.
25. Wakai A, et al. Cochrane Database Syst Rev. May 18 2017;5:CD009781.
26. Faktorovich EG, Melwani K. J Cataract Refract Surg. Oct 2014;40(10):1716-1730.
27. Flach AJ. Trans Am Ophthalmol Soc. 2001;99:205-210; discussion 210-202.
28. Asai T, et al., Cornea. Feb 2006;25(2):224-227.
29. Guidera AC, Luchs JI, Udell IJ. Ophthalmology. May 2001;108(5):936-944.
30. Pelino ASGCJ. Clinical Eye and Vision Care 1996;8(1):25-35.
31. Barbariga M, et al., Invest Ophthalmol Vis Sci. Mar 1 2018;59(3):1305-1312.
32 Dionne, R. A. et al. Clin Pharmacol Ther 64, 562-568 (1998).
33 Jones, J. D. et al. Am J Drug Alcohol Abuse 39, 86-91, (2013).
34 Sindrup, S. H., Graf, A. & Sfikas, N. Eur J Pain 10, 567-571 (2006).
35 Hill, R. Trends Pharmacol Sci 21, 244-246, (2000).
36. Farazifard R, et al., Brain Res Brain Res Protoc. Dec 2005;16(1-3):44-49.
37. Ferrari G, et al., J Cataract Refract Surg. Apr 2013;39(4):638-641.
38. Molina-Ortega, et al. (2014) Man Ther 19: 411-417;
39. Muñoz, Coveñas (2013) Peptides; 48:1-9.
40. Huan et al (2010) Expert Opinion therapeutic patents 20(8): 1019-1045
41. Walpole et al, British Journal of Pharmacology (1998); 124:83-92
42. Hale JJ et al, J Med Chem 1998; 41 (23) 4607-14
43. Hagiwara D et al, J Med Chem 1994; 37: 2090-9
44. Lowe JA et al. J Med Chem 1994 37:2831 - 40
45. Desai MC et al. J Med Chem 1992; 35:4911-3
46. Rosen TJ et al. Bioorg Med Chem Lett 1998; 8:281-4,
47. Paliwal S et al, Bioorg Med Chem Lett 2008; 18:4168-71,
48. Set S, et al. Bioorg Med Chem II,Kett 2005; 15:1479-84
49. Seto S, et al. Bioorg Med Chem Lett 2005; 15:1479-84
50. Nema S et al., PDA J Pharm Sci Technol. 1997 Jul-Aug;51(4):166-71.
51. Martinsen A., et al., Biotech. & Bioeng., 33 (1989) 79-89

## Claims

1. An NK-1 antagonist for use in the prevention and/or the treatment of ocular sensitivity and/or ocular pain.

2. The NK-1 antagonist for use according to claim 1, wherein the NK-1 antagonist is Fosaprepitant or a pharmaceutically acceptable salt thereof.

3. The NK-1 antagonist for use according to claim 1 or 2, wherein the NK-1 antagonist is administered at a concentration of at least 0.1 mg/ml, preferably of at least 1 mg/ml, preferably at least 10 mg/mL, preferably of at least 20 mg/mL, preferably of at least 30 mg/mL, preferably of at least 40 mg/mL, preferably of at least 50 mg/mL, preferably of at least 60 mg/mL, preferably of at least 100 mg/ml.

4. The NK-1 antagonist for use according to anyone of claims 1-3, wherein the NK-1 antagonist is administered once at a concentration of approximately 10 mg/mL.

5. The NK-1 antagonist for use according to anyone of claims 1-4, wherein the NK-1 antagonist is administered between once and six times a day.

6. The NK-1 antagonist for use according to anyone of claims 1-5, wherein the NK-1 antagonist is administered for 1 to 30 days, preferably at a concentration of 1 to 100 mg/ml, preferably at a concentration of 10 mg/ml.

7. The NK-1 antagonist for use according to anyone of claims 1-6 wherein the ocular sensitivity and/or ocular pain is caused by any one of the following conditions or pathology: keratitis, conjunctivitis, blepharitis, uveitis, corneal edema, dry eye, bullous keratopathy, ocular trauma/injuries, photo refractive keratotomy, radial keratotomy, contact lens intolerance, glaucoma, inflammatory disorders such as ocular pemphigoid, atopic conjunctivitis, rosacea, graft rejection, Lyell's syndrome, Stevens-Johnson syndrome, graft versus host disease; infectious keratitis including viral keratitis such as keratitis caused by infection with herpes simplex or herpes zoster, viral interstitial keratitis, bacterial keratitis such as keratitis caused by infection with Pseudomonas (e.g. Pseudomonas Aeruginosa), *Chlamydia trachomatis,* Treponema pallidum), fungal keratitis such as keratitis caused by Candida, Fusarium and Aspergillus spp and parasitic keratitis such as keratits caused by Onchocerciasi; degenerative disorders including congenital disorders such as pterygium, Terrien's marginal degeneration and aniridia; traumatic disorders such as ulcerations, acid burns, alkali burns; trauma and/or ocular tissue disruption associated with medical or surgical procedures; disorders associated with extended contact lens wear; stem cell deficiency (e.g. of limbus), including idiopathic, traumatic, aniridia, autoimmune polyendocrinopathy, infections caused by *staphylococcus, streptococcus, Pseudomonas* or microbial keratoconjuctivitis, *Pseudomonas aeuriginosa* infection, chemical or physical insult of the eye, ocular surgery, minor ocular procedures, refractive surgery, corneal crosslinking, cataract surgery, keratoplasty, glaucoma surgery, retina surgery, Goldman tonometry, gonioscopy, contact lens application with the three mirror lens, laser photocoagulation of the retina and any procedure inducing temporary or permanent stimulation of the trigeminal nerve fibers reaching the eye.

8. The NK-1 antagonist for use according to anyone of claims 1-7, wherein the NK-1 antagonist is administered prior to a surgery to the eye.

9. A pharmaceutical composition comprising an NK-1 antagonist and a pharmaceutically acceptable vehicle, for use in the prevention and/or the treatment of ocular sensitivity and/or ocular pain.

10. The pharmaceutical composition for use according to claim 9, wherein the NK-1 antagonist is Fosaprepitant or a pharmaceutically acceptable salt thereof.

11. The pharmaceutical composition for use according to claim 9 or 10, comprising 0.1 ng/ml to 10 mg/ml, preferably 100 ng/ml to 10 mg/ml, such as 100 µg/ml to 10 mg/ml, preferably 1 mg/ml to 10 mg/ml NK-1 antagonist or preferably 100 µg/ml NK-1 antagonist.

12. The pharmaceutical composition for use according to claim 9 or 10, comprising at least 1 mg/mL of NK-1 antagonist, preferably at least 10 mg/mL of NK-1 antagonist.

13. The pharmaceutical composition for use according to any one of claims 9-12, further comprising at least one agent selected from the group consisting of: an anaesthetic agent, a non-steroidal anti-inflammatory agent, an analgesic agent, an agent useful in the prevention and/or treatment of the disease or condition that causes the ocular sensitivity and/or ocular pain, and an agent that is used following surgery to the eye.

14. The pharmaceutical composition for use according to any one of claims 9 to 13 for topical use.

15. Eye-drops comprising an NK-1 antagonist and a pharmaceutically acceptable vehicle, for use in the prevention and/or the treatment of ocular sensitivity and/or ocular pain.
